# FASCICULE DE BREVET EUROPEEN

(11) **EP 0 719 265 B1**
(45) Date de publication et mention de la délivrance du brevet: **02.12.1998**
(21) Numéro de dépôt: 94926977.3
(22) Date de dépôt: 12.09.1994
(51) Int. Cl.: C07D 413/04, C07D 473/34

(54) **DERIVES DE NUCLEOSIDES, PROCEDES DE FABRICATION DE CES DERIVES DE NUCLEOSIDES ET ANTICORPS POLYCLONAUX ET MONOCLONAUX SPECIFIQUES DE CES DERIVES**
NUCLEOSID DERIVATE, VERFAHREN ZU IHRER HERSTELLUNG UND FÜR DIESE DERIVATE SPEZIFISCHE MONOKLONALE UND POLYKLONALE ANTIKÖRPER
NUCLEOSIDE DERIVATIVES, METHODS FOR THE MANUFACTURE THEREOF AND SPECIFIC POLYCLONAL AND MONOCLONAL ANTIBODIES OF SAID DERIVATIVES

(30) Priorité: 13.09.1993 FR 9310864
(43) Date de publication de la demande: 03.07.1996
(73) Titulaire: COMMISSARIAT A L'ENERGIE ATOMIQUE, 75015 Paris (FR); CENTRE NATIONAL D'ETUDES SPATIALES, 75001 Paris (FR)
(72) Inventeur: MOLKO, Didier, F-38210 Tullins (FR); CADET, Jean, F-73160 Cognin (FR); CIMAZ, Isabelle, F-38000 Grenoble (FR)
(74) Mandataire: Des Termes, Monique
(86) Numéro de dépôt international: FR9401070
(87) Numéro de publication internationale: WO9507907

(56) Documents cités:
- WO-A-91/18898
- WO-A-92/05186
- US-A- 4 515 781
- CHEMICAL ABSTRACTS, vol. 103, no. 25, 23 Décembre 1985, Columbus, Ohio, US; abstract no. 210401c, RAYFORD RICHARD ET AL. 'Reductive alkylation with oxidized nucleotides...' page 478 ;colonne 2 ;
- PROCEEDINGS OF THE NATIONAL ACADEMY OF SCIENCES OF THE UNITED STATES OF AMERICA, vol.52, no.1, Juillet 1964 pages 68 - 74 BERNARD F. ERLANGER ET AL. 'Antibodies specific for Ribonucleosides and Ribonucleotides.....' cité dans la demande
- CARCINOGENESIS, vol.12, no.5, 1991 pages 865 - 871 PAOLO DEGAN ET AL. cité dans la demande
- PROGRESS IN NUCLEIC ACID RESEARCH AND MOLECULAR BIOLOGY , ACADEMIC PRESS, vol.42, 1992 pages 39 - 77 B. DAVID STOLLAR 'Immunochemical Analyses of Nucleic Acids' cité dans la demande
- MUTATION RESEARCH, ELSEVIER, vol.233, 1990 pages 219 - 233 CHRISTOPHER P. WILD 'Antibodies to DNA alkylation adducts as analytical tools in chemical carcinogenesis' cité dans la demande

## Description

L'invention concerne des dérivés de nucléosides, des procédés de fabrication de ces dérivés de nucléosides, ainsi que des anticorps polyclonaux et monoclonaux spécifiques des dérivés précités.

La macro molécule d'A.D.N. (acide désoxyribonucléique) est le constituant des chromosomes et les différents segments de cette molécule forment les gènes, supports des caractères héréditaires. L'A.D.N. se présente sous la forme d'une double hélice spiralée, formée alternativement de sucre (désoxyribose) et de phosphate, les spirales des deux chaînes étant réunies localement par des groupements de bases nucléiques azotées, de type purinique ou pyrimidinique. Les nucléotides constituant l'A.D.N. sont les esters phosphoriques des nucléosides.

Les bases nucléiques de l'A.D.N. d'un individu (ou d'un animal ou d'un végétal) peuvent être modifiées et endommagées lorsque cet individu est exposé à un rayonnement solaire intense, au rayonnement cosmique (vols intercontinentaux), à des photosensibilisateurs, au contact avec l'amiante ou à une irradiation ionisante, que celle-ci soit accidentelle ou due à un traitement de radiothérapie. Ces modifications des bases nucléiques de l'A.D.N. pouvant entraîner une modification importante du patrimoine génétique de l'individu concerné, il est particulièrement important de détecter si de telles modifications se sont produites et de préciser la nature des modifications survenues.

Il serait donc intéressant de mettre au point une technique de dosage susceptible d'être utilisée chez les patients suspectés d'avoir subi des modifications de leur A.D.N.

Parmi les diverses techniques de dosage permettant de déterminer la présence d'A.D.N. modifié dans un échantillon, les immunodosages consistent à faire réagir un anticorps spécifique d'une modification particulière de l'A.D.N avec un échantillon contenant de l'A.D.N isolé ou hydrolysé. Ces anticorps sont généralement produits par clonage.

La fabrication d'anticorps polyclonaux (ou monoclonaux) nécessite tout d'abord la fabrication d'antigènes spécifiques, c'est-à-dire de nucléosides ou de nucléotides dont les bases nucléiques ont subi les modifications que l'on cherche à détecter, ces nucléosides ou nucléotides étant liés à une grosse molécule, par exemple une protéine. Un nucléoside ou un nucléotide seul trop petit n'est pas perçu par le système immunitaire. Ensuite, les anticorps polyclonaux sont produits par un mammifère qui a reçu une injection de l'antigène précité, cet antigène comprenant une protéine étrangère au mammifère considéré, liée à un haptène (molécule de petite taille contre laquelle on souhaite obtenir les anticorps spécifiques).

On connaît déjà d'après l'art antérieur des techniques de dosages immunologiques des acides nucléiques. L'article de Christopher P. WILD : "Antibodies to D.N.A. alkylation adducts as analytical tools in chemical carcinogenesis", Mut. Res., (1990), 233, 219-233, est justement consacré aux anticorps spécifiques de nucléotides dont les bases nucléiques ont subi des modifications par alkylation. Cet auteur insiste sur le rôle des anticorps dans les dosages immunologiques utilisés dans les études épidémiologiques des cancers humains et de la cancérogénèse chimique, due notamment à des agents alkylants.

L'article de B. D. Stollar : "Immunochemical Analyses of nucleic acids", Progress in Nucleic Acid, Research and Molecular Biology, (1992), 42, 39-75, concerne également des anticorps spécifiques des acides nucléiques.

Par ailleurs, certains défauts oxydatifs de l'A.D.N. ont fait l'objet de plusieurs publications.

Ainsi, l'article de G.J. West et al. ; "Radioimmunoassay of 8-hydroxyadenine", Int. J. Rad. Biol., (1982), 42, 481-490, décrit des dosages radio-immunologiques de la 8-hydroxyadénine. Cette modification de l'A.D.N. peut survenir après une irradiation aux rayons.

L'article de P. Degan et al., "Quantitation of 8-hydroxy-2'-deoxyguanosine in DNA by polyclonalantibodies", Carcinogenesis, (1991), 12, 865-871, décrit des anticorps polyclonaux spécifiques de la 8-hydroxy-2'-désoxyguanosine et de la 8-hydroguanine. Ces anticorps polyclonaux peuvent être utilisés dans des dosages immunologiques afin d'isoler rapidement les deux types précités de guanosine modifiée, dans un échantillon d'urine par exemple.

L'article de H.L. Lewis et al., "Serologic Assay of DNA Base Damage", Rad. Res. (1978), 75, 305-316, concerne la préparation d'anticorps polyclonaux et le dosage de la 5-hydroxyméthyldésoxyuridine, obtenue après irradiation ionisante de la thymidine.

Enfin, l'article de S.A. Leadon et P.C. Hanawalt, "Monoclonal antibody to DNA containing thymine glycol", Mut. Res., (1983), 112, 191-200, concerne des anticorps monoclonaux de la 5,6-dihydroxy-5,6-dihydrothymine (thymine glycol) obtenue après que de l'A.D.N. ait été soumis à des radiations ionisantes ou proches de l'ultraviolet. Ces anticorps monoclonaux ont été obtenus en faisant fusionner des cellules de miélome de souris avec des cellules de rate provenant de la lignée de souris BALB-c, ces souris ayant été immunisées avec une poly(d-thymine) oxydée par OsO₄ puis complexée avec de la sérumalbumine de bovin méthylée. Les tests sont effectués par des méthodes ELISA.

L'article de B.F. Erlanger et S.M. Beiser, "Antibodies specific for ribonucleosides and ribonucleotides and their reaction with DNA", Proc. N.A.S. USA, (1964), 52, 68-74, décrit un protocole de couplage d'un acide nucléique avec une protéine porteuse, permettant ce former un antigène, susceptible d'être utilisé dans un protocole d'immunisation de lapins. Ce protocole de couplage est représenté ci-dessous. 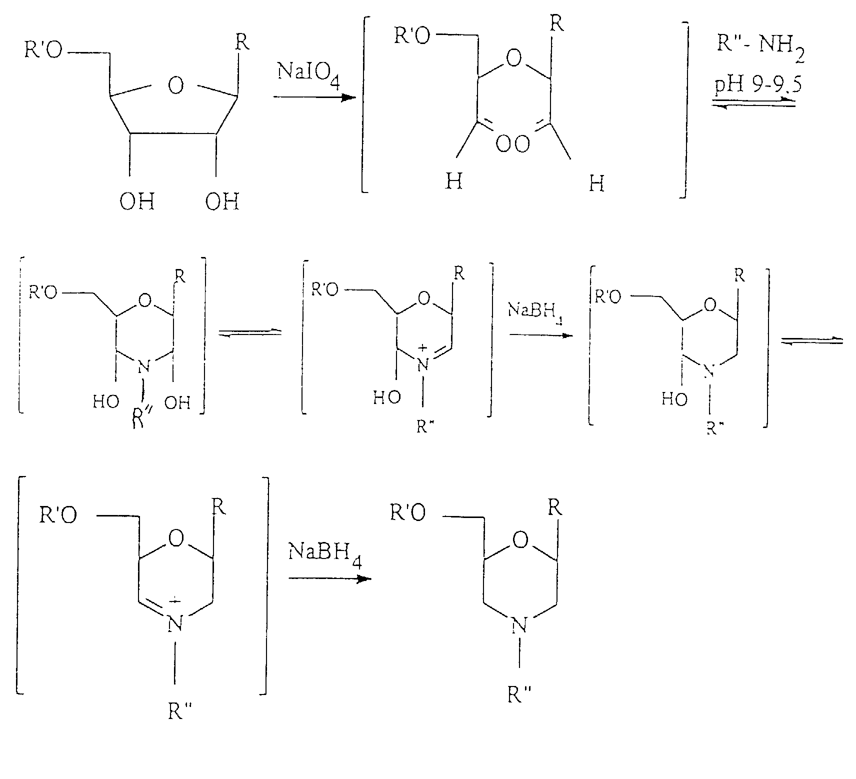 R représentant une base purinique ou pyrimidinique, R' représentant H ou -PO-(OH)₂ et R" représentant le reste d'une protéine.

Ce procédé consiste à agir sur l'ose d'un ribonucléoside par action du periodate de sodium. Le cycle de l'ose est ouvert entre les carbones 2' et 3' et il se forme un dialdéhyde. Ce dialdéhyde est alors couplé à une protéine, à un pH voisin de 9 à 9,5. Le NaBH₄ intervient pour réduire la base de Schiff obtenue comme produit intermédiaire.

Toutefois, le procédé divulgué dans cet article est limité à des bases nucléiques n'ayant pas subi de modifications. En effet, avec certaines bases fragiles sensibles à l'oxydation et/ou à la réduction, il serait impossible de modifier au préalable ces bases de nucléosides, puis de faire subir à ces nucléosides modifiés, une étape d'oxydation par le periodate de sodium, suivie d'une étape de réduction par le borohydrure de sodium. Après un tel traitement chimique, la plupart des défauts pour lesquels on souhaite étudier les A.D.N. modifiés, seraient altérés.

En outre, l'enchaînement des réactions décrit dans ce procédé est effectué sans isoler les produits intermédiaires. En conséquence, un certain nombre de produits parasites sont susceptibles d'apparaître au cours du procédé et d'être présents dans la protéine conjuguée finale. Chaque produit parasite peut potentiellement induire une réaction immunitaire qui lui est propre. Dans ce cas, le mélange d'anticorps obtenu pourrait n'avoir qu'une faible sélectivité. Ceci est le cas dans l'article de H.L. Lewis et al. précédemment cité, où la molécule cible était la 5-hydroxyméthyluracile et où l'anti-sérum obtenu reconnaissait également la thymine qui est une base naturelle de l'A.D.N.

On connaît également d'après l'article de T. Okabayashi et al., "A radioimmunoassay for 1-β -D-Arabinofuranosylcytosine", Cancer Res., (1977), 17, 619-624, un procédé consistant à faire réagir un dérivé de l'acide succinique avec un désoxynucléoside dont la base a été modifiée, puis à créer une fonction amide avec l'amine libre d'une protéine. Ce procédé est illustré ci-dessous. 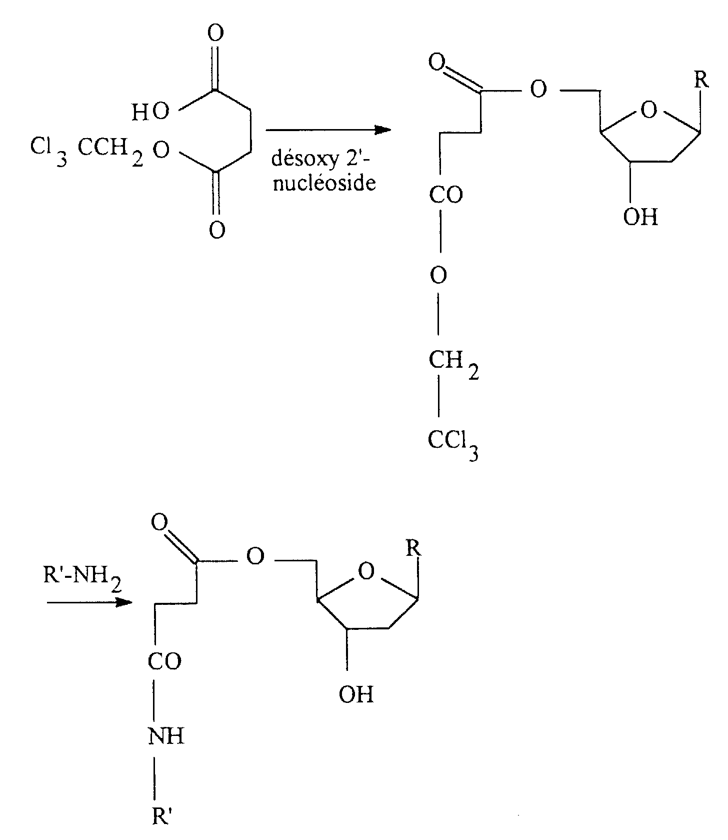 R représentant une base purinique ou pyrimidinique et R' le reste d'une protéine.

Le principal défaut de ce procédé réside dans le manque de stabilité de la fonction ester utilisée pour conjuguer ie nucléoside à la protéine.

Enfin, on connaît également d'après un article de M.D. Friesen et al., "Isolation of Urinary 3-methyladenine", Chem. Res. Toxicol., (1991), 4, 102-106, l'une des méthodes les plus employées pour coupler une base nucléique alkylée à une protéine. Cette méthode est illustrée ci-dessous. 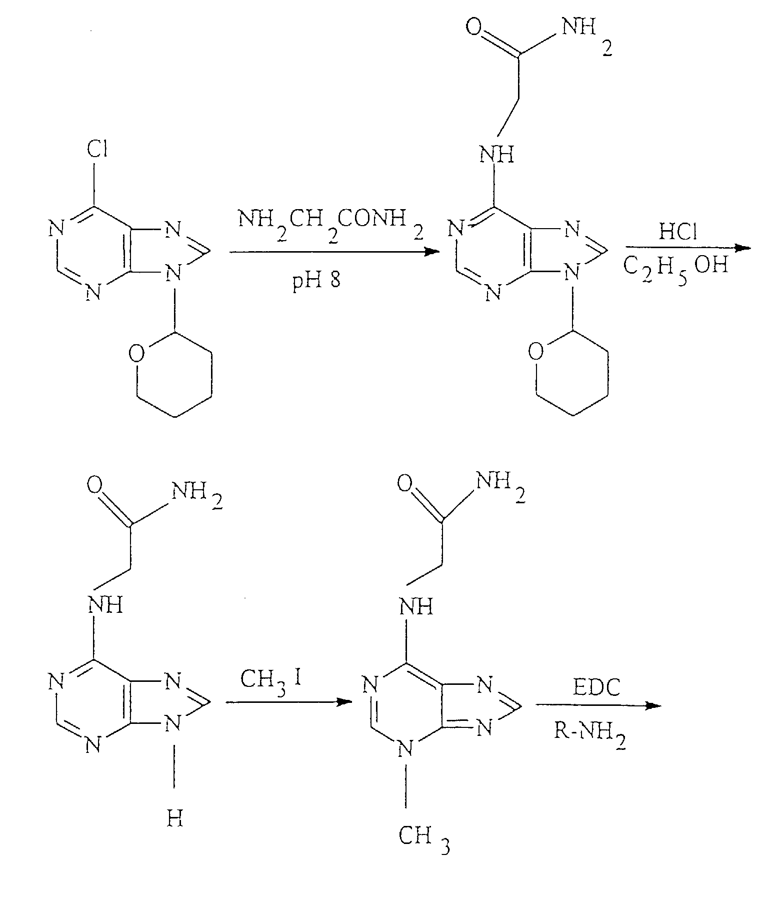 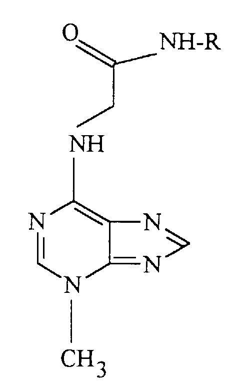 dans laquelle R est une protéine et EDC représente le chlorhydrure de 1-[3-diméthylamino)propyl]-3-éthyldicarbodiimide.

Cette méthode diffère des deux précédentes en ce que le produit de départ n'est ni un ribo ni un désoxyribonucléoside modifié, mais un dérivé alkylé de la base. Elle permet de former des anticorps d'une excellente qualité car l'haptène utilisé est purifié juste avant sa conjuguaison à la protéine. Toutefois, cette technique est plus difficile à mettre en oeuvre que les deux procédés exposés précédemment.

L'invention a consisté à mettre au point des antigènes et des procédés de fabrication de ces antigènes évitant les inconvénients précédemment cités.

A cet effet, l'invention concerne des dérivés de nucléosides caractérisés en ce qu'ils répondent à la formule chimique suivante : 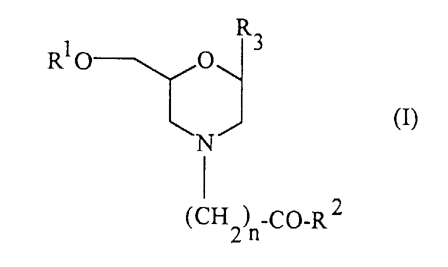 dans laquelle n représente un nombre entier positif quelconque, R¹ représente H ou un acide mono, di ou tri-phosphorique linéaire, R² représente un groupe hydroxyle, un groupe alkyloxy, un groupe aryloxy, une protéine comprenant un site aminé, un aminoalkyl polystyrène ou une silice greffée avec une chaîne alkylamine et R³ représente une base nucléique modifiée choisie parmi l'uracile, la thymine, la cytosine, la guanine ou l'adénine modifiée.

On utilise l'expression "dérivés de nucléosides" puisqu'ils sont formés à partir de nucléosides, toutefois dans ces dérivés l'ose a disparu et est remplacé par la morpholine.

Lorsque R² représente une protéine, l'antigène obtenu peut servir de base à la fabrication d'anticorps.

Lorsque R² est un aminoalkylpolystyrène ou une silice greffée, par exemple, le produit obtenu est un support solide auquel est lié l'haptène. De tels supports peuvent être utilisés dans des dosages de type ELISA (marque déposée).

L'invention concerne également un procédé de fabrication de dérivés de nucléosides répondant à la formule chimique suivante : 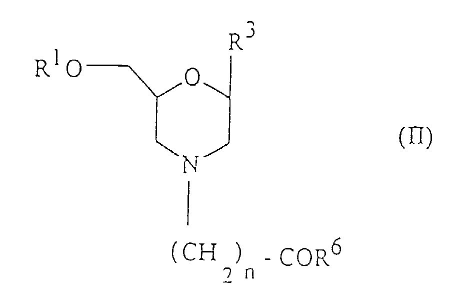 dans laquelle n représente un nombre entier positif quelconque, R¹ représente H ou un acide mono, di ou tri-phosphorique linéaire, R⁶ représente un groupe hydroxyle, un groupe alkyloxy ou un groupe aryloxy et R³ représente une base nucléique modifiée choisie parmi l'uracile, la thymine, la cytosine, la guanine ou l'adénine modifiée, consistant à modifier un dérivé de nucléoside répondant à la formule chimique suivante : 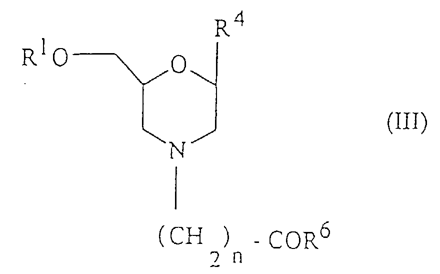 dans laquelle n représente un nombre entier positif quelconque, R¹ et R⁶ ont la même signification que précédemment et R⁴ représente une base nucléique choisie parmi l'uracile, la thymine, la cytosine, la guanine ou l'adénine, au moyen d'un agent substituant.

Contrairement à certains procédés connus de l'art antérieur, le procédé selon l'invention ne fait pas intervenir de liaison ester fragile entre le nucléoside modifié et la protéine. Les conjugués obtenus sont donc beaucoup plus stables et peuvent être stockés en solution sans être dégradés, pendant une période plus longue.

Le produit de départ (III) présente une morpholine à la place du cycle désoxyribosidique. Ce produit est stable. Il est en outre soigneusement purifié avant d'être utilisé dans le procédé. Ceci évite la présence de "parasites" ou contaminants dans l'haptène fabriqué.

Enfin, le produit de départ (III) a déjà subi la transformation du cycle osidique en cycle morpholinique avant que la base nucléique ne soit modifiée au cours du procédé selon l'invention. Cela permet d'envisager la préparation d'antigènes dans lesquels les bases nucléiques sont oxydées ou réduites, ce qui est beaucoup plus difficile à obtenir si la synthèse du cycle morpholinique intervient après la modification de la base nucléique.

L'invention concerne également un autre procédé de fabrication de dérivés de nucléosides répondant à la formule chimique suivante : 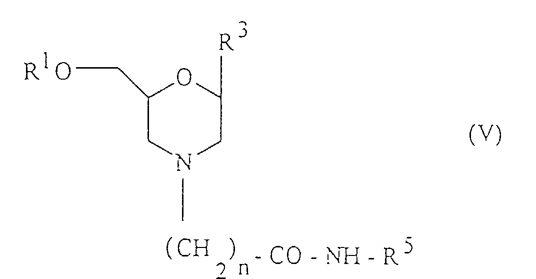 dans laquelle n représente un nombre entier positif quelconque, R¹ représente H ou un acide mono, di ou triphosphorique linéaire, R³ représente une base nucléique modifiée choisie parmi l'uracile, la thymine, la cytosine, la guanine ou l'adénine modifiée et R⁵ représente le reste d'une protéine, un alkylpolystyrène ou une silice greffée avec une chaîne alkyle,
ce procédé consistant à faire réagir un composé du type NH₂-R⁵, dans lequel R⁵ a la même signification que précédemment avec un dérivé de nucléoside répondant à la formule chimique suivante : 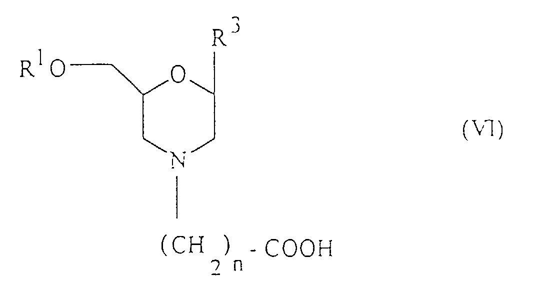 dans laquelle n, R¹ et R³ ont la même signification que précédemment.

Lorsque NH₂-R⁵ est une protéine, le produit (V) obtenu est un antigène. Lorsque NH₂-R⁵ est un aminoalkylpolystyrène ou une silice greffée avec une chaîne alkylamine, le produit (V) obtenu est un support solide. Ainsi, dans un procédé de dosage ELISA, les parois des tubes à essais ou les puits des plaques pourraient être recouverts ce silice greffée, ce qui pourrait permettre de lier l'haptène au support de manière covalente.

Par ailleurs, la Société Pharmacia commercialise un appareil destiné à étudier de manière dynamique les interactions entre molécules biologiques (connu sous la marque déposée BiaCore) . Le principe consiste à fixer une molécule sur un support solide et à faire circuler une solution contenant l'autre protagoniste. Le support solide obtenu selon l'invention peut être utilisé dans ce type d'appareil.

Par ailleurs, l'invention concerne également des anticorps polyclonaux anti-dérivé de nucléosides, obtenus par immunisation d'un mammifère appropriée avec l'antigène répondant à la formule chimique suivante : 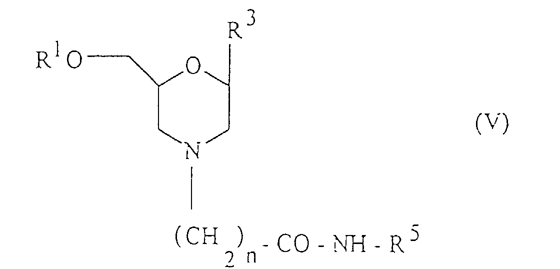 dans laquelle n représente un nombre entier positif quelconque, R¹ représente H ou un acide mono, di ou triphosphorique linéaire, R³ représente une base nucléique modifiée choisie parmi l'uracile, la thymine, la cytosine, la guanine ou l'adénine modifiée, et R⁵ représente une protéine ne provenant pas dudit mammifère.

Enfin, l'invention concerne des anticorps monoclonaux anti-dérivés de nucléosides obtenus en faisant fusionner des cellules de myélome d'un mammifère avec des cellules spléniques de souris immunisées par un antigène répondant à la formule chimique suivante : 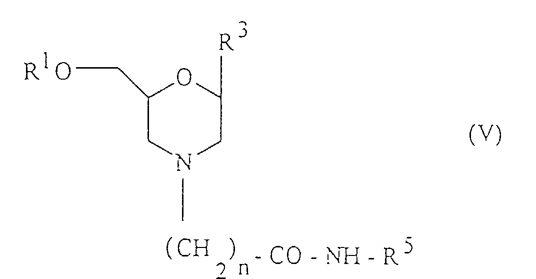 dans laquelle n représente un nombre entier positif quelconque, R¹ représente H ou un acide mono, di ou triphosphorique linéaire, R³ représente une base nucléique modifiée choisie parmi l'uracile, la thymine, la cytosine, la guanine ou l'adénine modifiée, et R⁵ représente une protéine ne provenant pas de la souris.

L'invention sera mieux comprise à la lecture de la description suivante d'un mode de réalisation de l'invention donné à titre d'exemple illustratif et non limitatif.

Un premier procédé de préparation des dérivés de nucléosides selon l'invention consiste à utiliser un nucléotide ou un nucléoside déjà modifié par formation d'une morpholine, de façon à pouvoir se lier ultérieurement par exemple avec un groupe aminé appartenant à une protéine.

Le produit de départ (III) (dénommé ci-après "morpholinonucléoside") présente la formule chimique suivante : 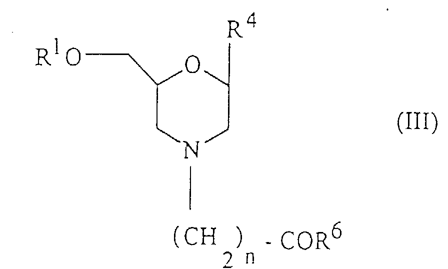 dans laquelle n représente un nombre entier positif quelconque, R¹ représente H ou acide mono, di ou tri-phosphorique linéaire, R⁶ représente un groupe hydroxyle, un groupe alkyloxy ou un groupe aryloxy et R⁴ représente une base nucléique choisie pari l'uracile, la thymine, la cytosine, la guanine ou l'adénine.

Ce produit (III) peut être obtenu par exemple selon un procédé décrit dans l'article de R. Rayford et al., "Reductive alkylation with oxydized nucleotides", J. Biol. Chem., (1985), 260, 15708-15713 et illustré ci-dessous : 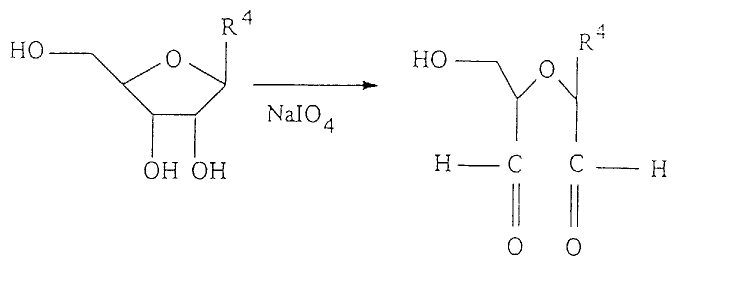 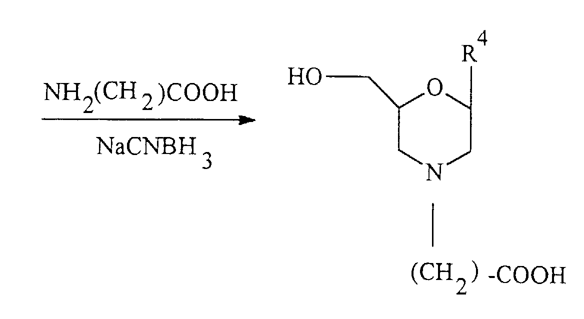 R⁴ ayant la même signification que précédemment.

Ce procédé consiste à faire réagir un nucléoside avec du periodate de sodium, afin d'ouvrir la liaison entre le carbone 2' et le carbone 3'. On obtient ainsi un dialdéhyde que l'on fait réagir avec du glycocolle pour former une double base de Schiff. On réduit cette double base par du NaCNBH₃ afin de conduire à un "morpholinonucléoside". Le nucléoside utilisé dans cet article était l'adénosine. Toutefois, de façon similaire, ce traitement peut être effectué avec d'autres bases puriniques ou pyrimidiniques. De même, le nucléoside peut être remplacé par un nucléotide dans lequel l'acide phosphorique est en position 5.

Lorsque R⁶ est un groupe alkyloxy ou un groupe aryloxy, le produit III est un ester. Il peut être obtenu par le procédé qui vient d'être décrit en remplaçant le glycocolle par un glycinate, tel qu'un glycinate d'éthyle ou de t-butyle, par exemple. De plus, ces dérivés peuvent être transformés en esters actifs pour être condensé sur une fonction amine.

Le produit de départ (III) du procédé selon l'invention peut également être obtenu par n'importe quel autre procédé.

Les agents substituants permettant d'effectuer les substitutions sur les bases puriniques ou pyrimidiniques, sont généralement des agents qui entraînent des modifications oxydatives de ces bases. Ces agents sont des agents chimiques ou un traitement physique. Les agents chimiques sont choisis par exemple parmi l'ozone, le peroxyde d'hydrogène, le brome combiné à la collidine, le brome et l'oxyde d'argent, le permanganate de potassium, le tétraoxyde d'osmium, le méthanal ou AlLiH₄. Le même résultat pourrait être obtenu par action de rayonnements ionisants en présence ou en l'absence d'oxygène, par photochimie (U.V. puis photosensibilisateur), par hydrogénation catalytique ou par traitement par le brome et l'eau puis hydrogénolyse. On donnera ci-après un tableau I récapitulatif des agents substituants et des substitutions qu'ils entraînent sur les différentes bases puriniques ou pyrimidiniques.

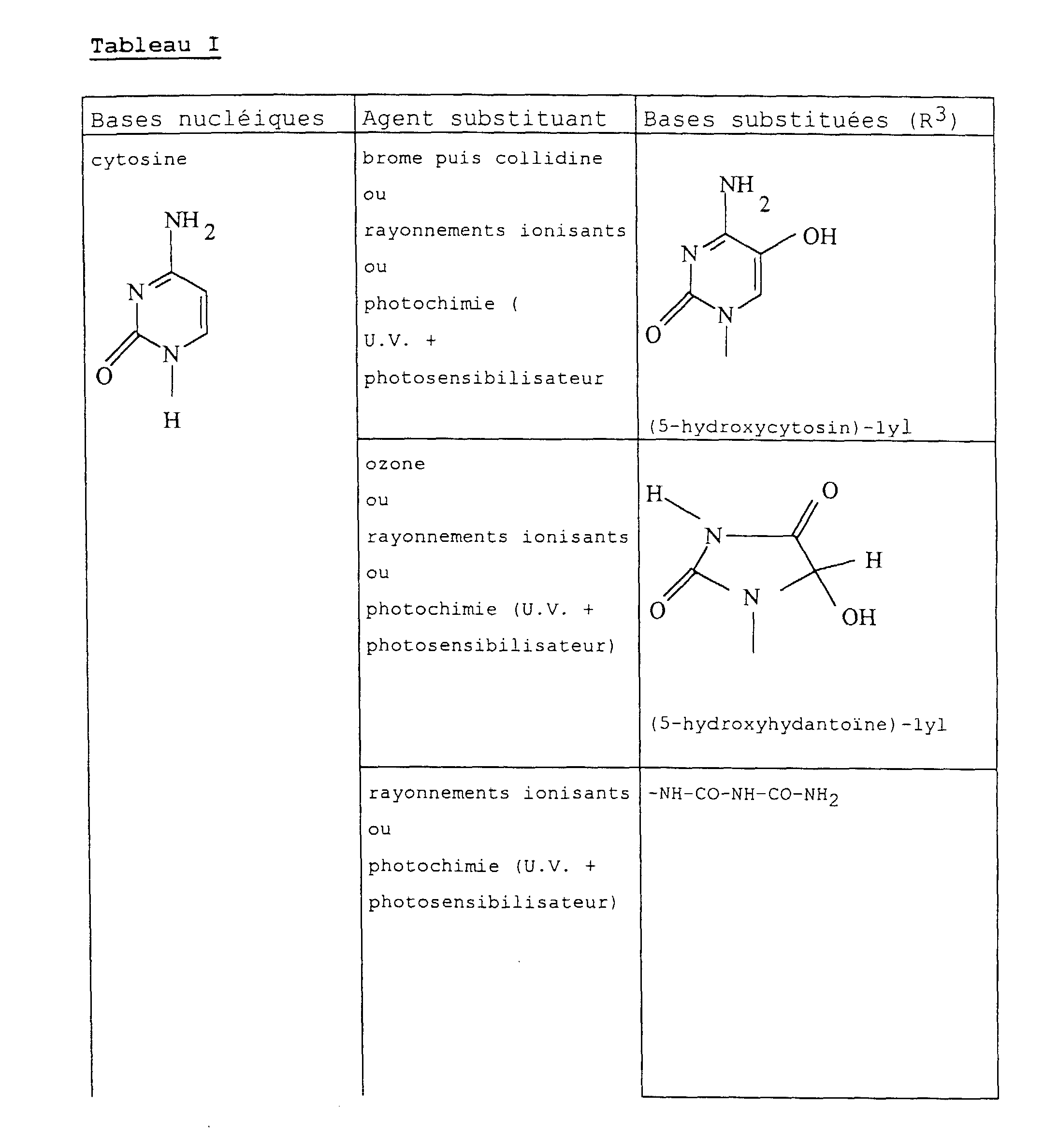

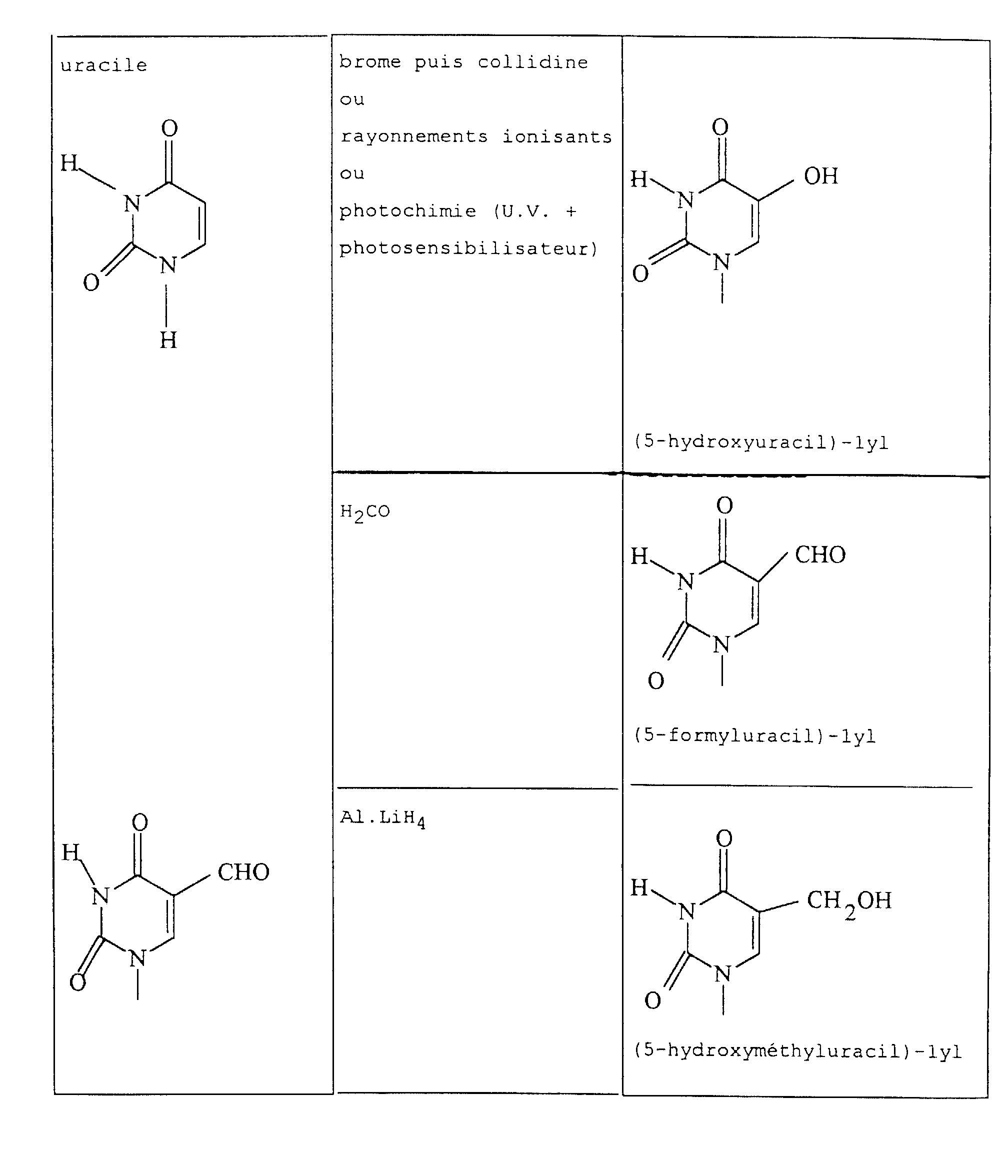

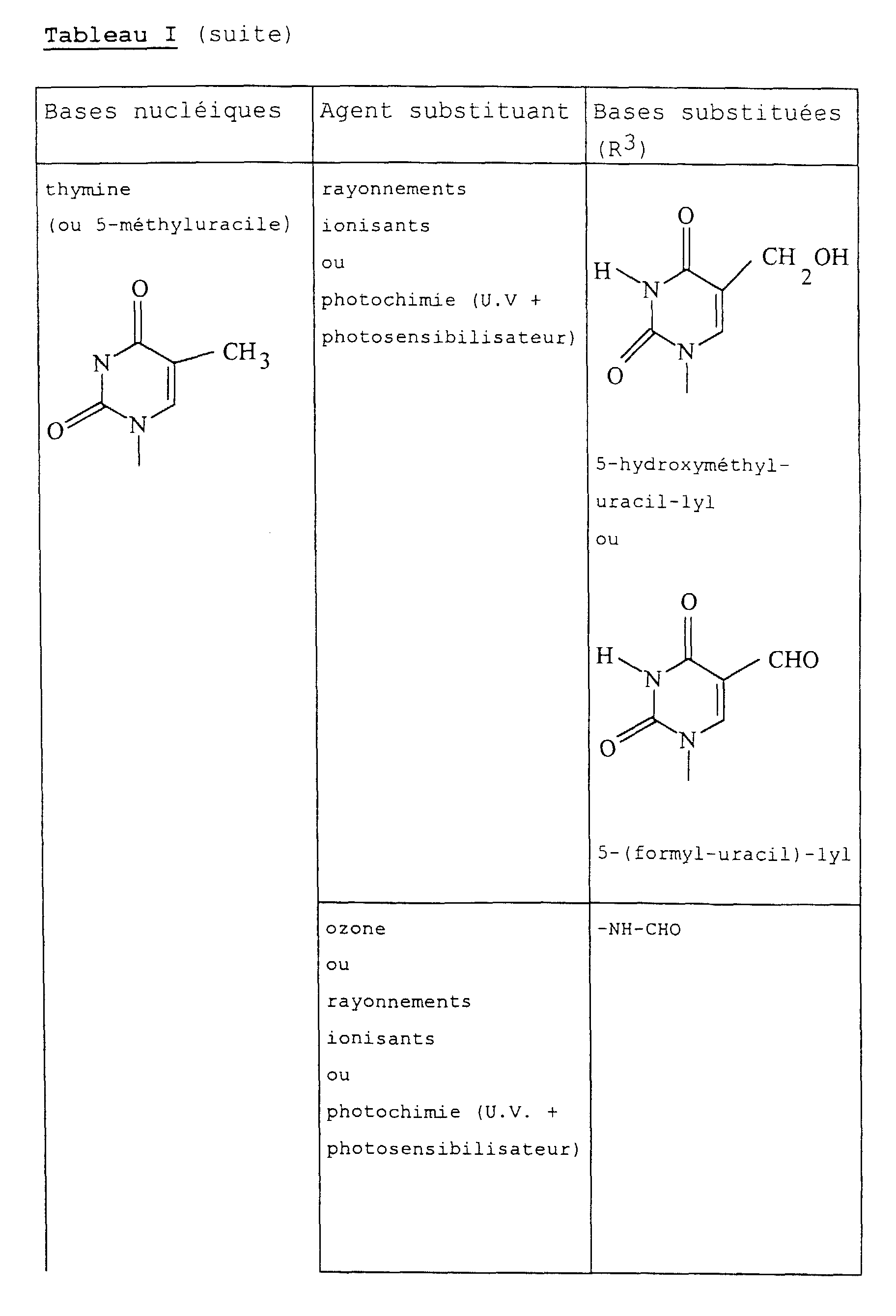

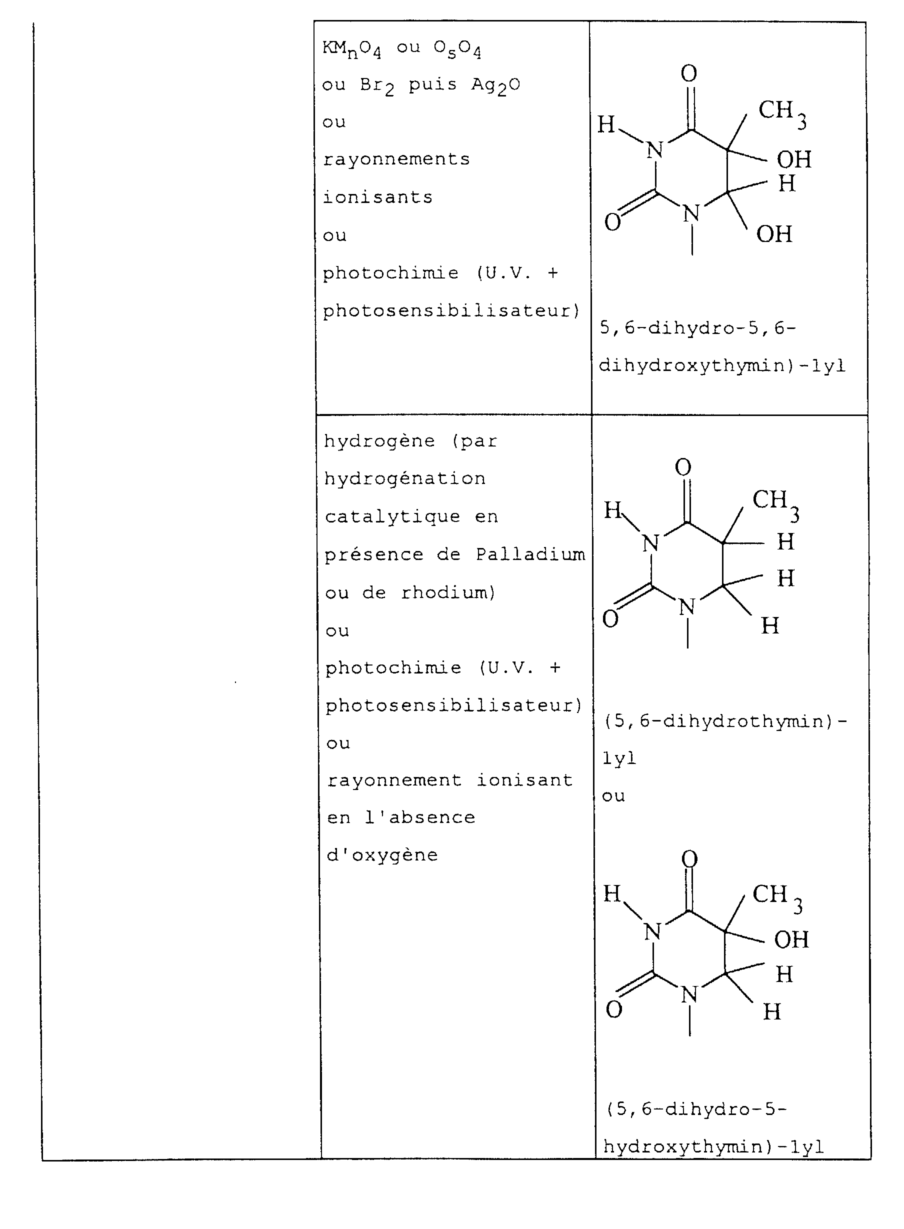

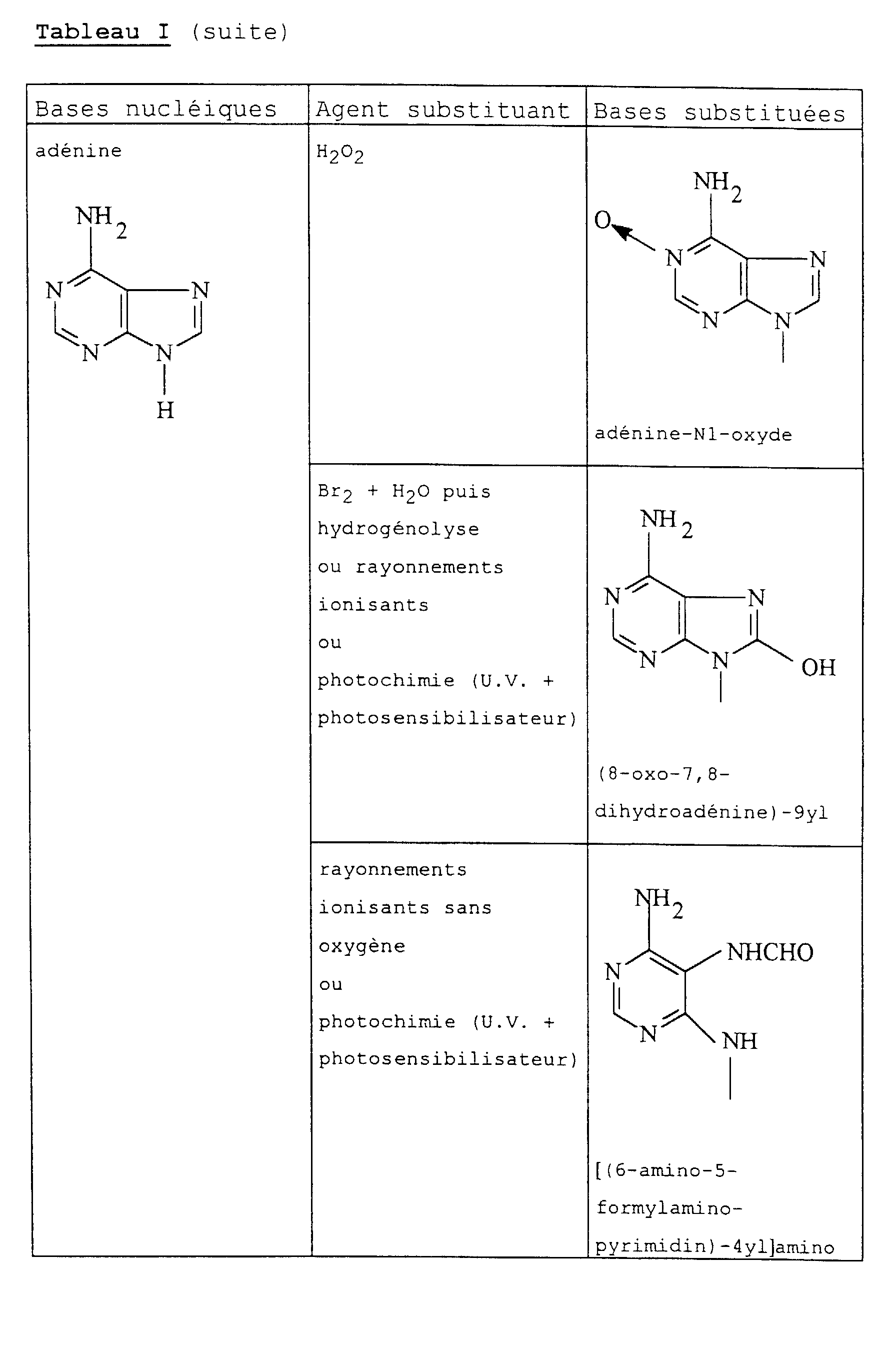

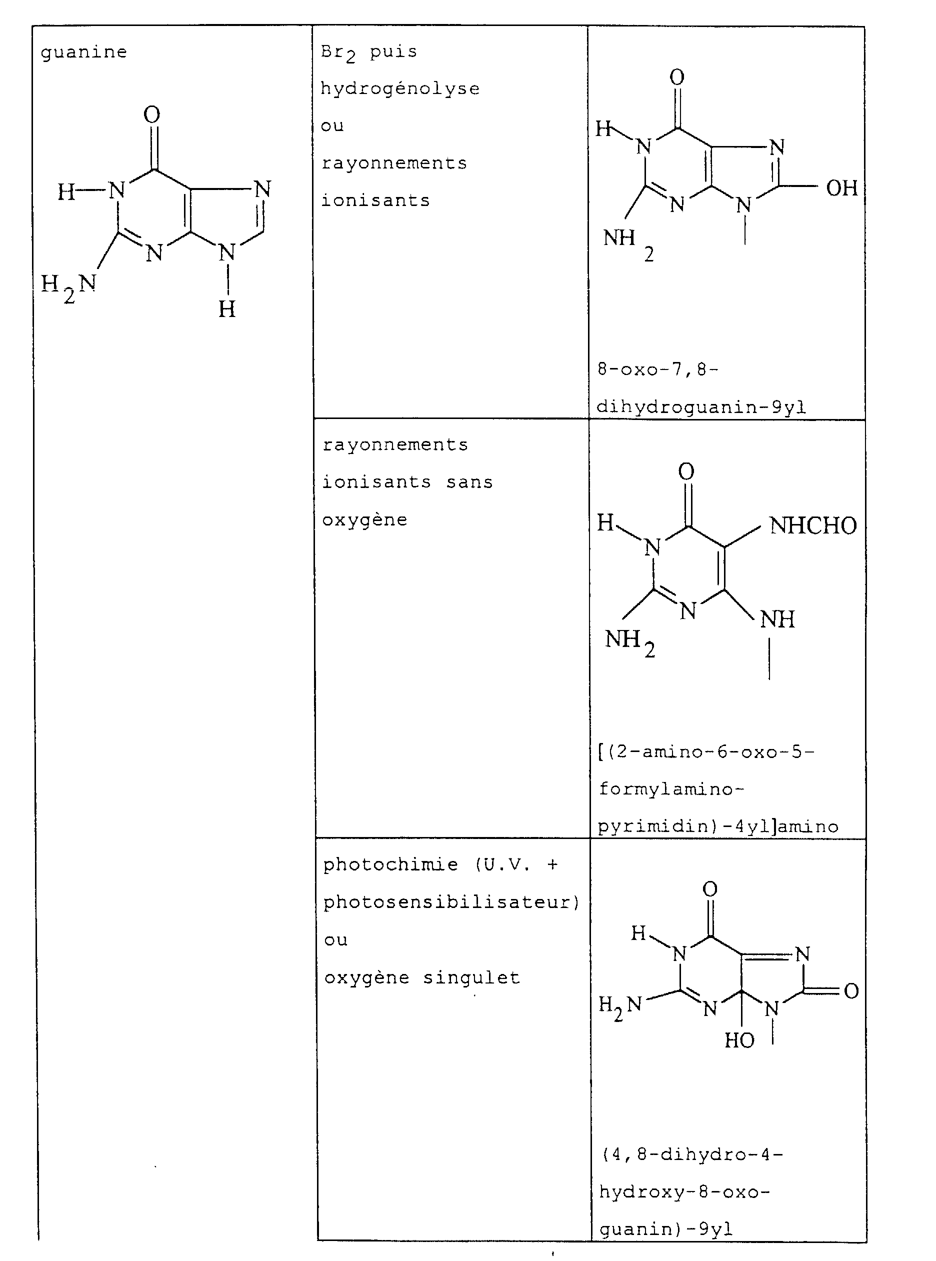

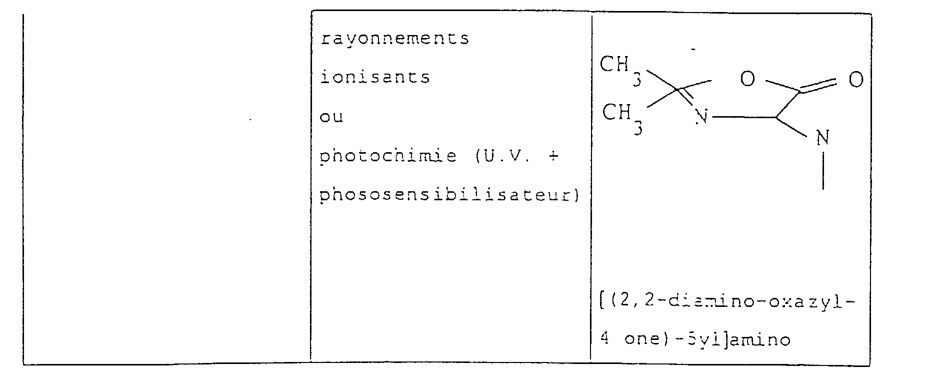

A l'issue du procédé selon l'invention, on obtient l'haptène de formule chimique suivante : 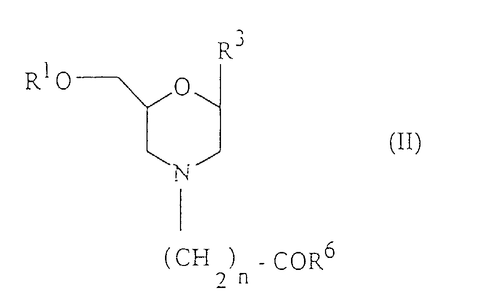 dans laquelle n représente un nombre entier positif quelconque, R¹ représente H ou un acide mono, di ou triphosphorique linéaire, R⁶ représente un groupe hydroxyle, un groupe alkyloxy ou un groupe aryloxy et R³ est une base nucléique modifiée choisie parmi la cytosine, l'uracile, la thymine, l'adénosine et la guanine modifiée et de préférence l'une des bases modifiées ou substituées du tableau I.

On notera que le produit II représente un certain nombre de variantes possibles du produit (I).

On donnera ci-après un exemple de préparation spécifique de l'un de ces haptènes.

### Exemple 1 : synthèse de la 2-(5-hydroxycytosine-1-yl)-4-carboxyméthyl-6-(hydroxyméthyl)-morpholine

On dissout 100mg de 2-(cytosine-1-yl)-4-carboxyméthyl-6-hydroxyméthyl morpholine dans 5 ml d'eau. On ajoute ensuite du brome goutte à goutte, jusqu'au maintien de la coloration jaune. Après 15 minutes d'agitation à température ambiante, l'excès de brome est chassé par barbotage d'air dans la solution. On ajoute ensuite 200 µl de collidine et la solution est agitée pendant 2 heures, à la température ordinaire. L'eau est évaporée sous pression réduite puis la collidine libre est éliminée par co-évaporation de 5 ml d'éthanol. Le résidu obtenu est analysé par chromatographie liquide haute pression (colonne Nucleosil 10-C18 de Macherey Nagel (marque déposée), dimensions 6x300 mm, phase mobile acétate de triéthylammonium 50 mM et méthanol). L'analyse montre la présence d'un produit majoritaire (60%) différent du composé de départ. L'analyse par RMN du produit collecté montre qu'il s'agit bien du composé attendu. Ce produit est caractérisé par un maximum d'absorption dans l'ultraviolet à 290,4 nm pour un coefficient d'extinction moléculaire de 5700 mol.l⁻¹. cm⁻¹.

Le deuxième procédé selon l'invention consiste à traiter l'haptène (VI) obtenu à l'issue du premier procédé de façon à former par exemple un antigène ou une phase solide (film, gel) dans laquelle l'haptène VI est fixé au support de manière covalente. Ce procédé est rappelé ci-dessous : 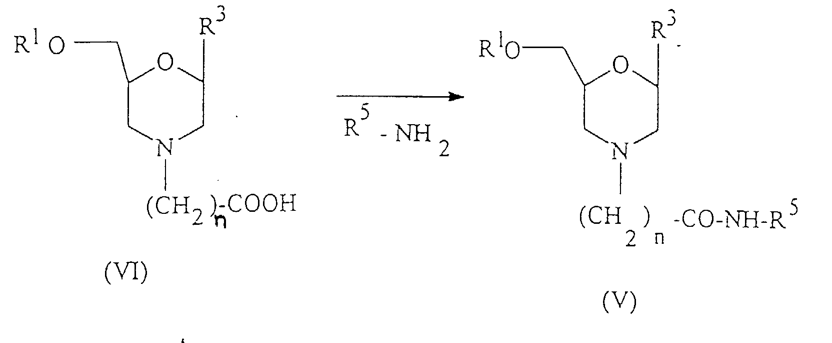 n, R¹ et R³ ont la même signification que précédemment et R⁵ représente le reste d'une protéine, un alkyl polystyrène ou une silice greffée avec une chaîne alkyle.

L'haptène de départ (VI) est purifié par chromatographie liquide à haute pression avant d'être couplé au radical NH₂-R⁵. Ce radical R⁵ peut être une protéine. Celle-ci est choisie par exemple parmi la sérum-albumine de bovin méthylée, l'ovalbumine de dinde ou de poulet ou les hémocyanines et notamment la KLH correspondant au terme anglais "keyhole limpet hémocianin", c'est-à-dire une protéine du coquillage connu sous le nom de bernique. On obtient alors un antigène susceptible d'être utilisé dans la fabrication d'anticorps. Lors de la fabrication d'anticorps polyclonaux, la protéine sera choisie de façon à être d'une nature différence de celle de l'animal utilisé pour la production d'anticorps. Les protéines précitées sont bien adaptées lorsque l'on souhaite immuniser un lapin.

Le radical NH₂-R⁵ peur être également un aminoalkylpolystyrène ou une silice greffée avec une chaîne alkylaminée. On obtient alors un film ou un gel dans lequel l'haptène est lié de manière covalente au support.

On notera que le produit (V) représente un certain nombre des variantes possibles du produit (I).

On donnera ci-après un exemple de réalisation de ce procédé.

### Exemple 2 : Synthèse d'un conjugué haptène-protéine (antigène) :

On dissout 30mg (90 µmoles) de 2-(5-hydroxycytosine-1-yl-)-4-carboxyméthyl-6-hydroxyméthylmorpholine dans 2 ml d'eau. Ce composé dissous dans l'eau est ajouté, en 3 heures, à 5 ml d'une solution contenant 35 mg (180 µmoles) de E.D.C. (N'-(3-diméthylaminopropyl)-N-éthylcarbodiimide(chlohydrate)) et 80 mg de sérummalbumine de bovin. Après avoir laissé reposer cette solution pendant une nuit à température ambiante, l'eau est évaporée sous pression réduite et le résidu obtenu est dissous dans 2 ml d'eau et analysé par chromatographie d'exclusion, (colonne Fractogel TSK-HW40 de Merck (marque déposée), dimensions 20x400 mm, phase mobile NaCl 0,15 M).

Le produit élué en premier est collecté, dialysé contre de l'eau et la solution obtenue est alors lyophilisée. On mesure alors la charge du produit en haptène. Cette mesure est obtenue en comparant le spectre d'absorption U.V. de la protéine d'origine à celui de la protéine greffée sur le nucléoside modifié. La comparaison de l'absorption à 270 et 300 nm permet de déterminer que 7,8 moles de 2-(5-hydroxycytosin-1-yl)-4-carboxyméthyl-6-hydroxyméthylmorpholine sont greffés par mole de protéine.

L'invention concerne enfin les anticorps spécifiques des antigènes (V) obtenus par le procédé précédent, dans lequel R⁵ représente le reste d'une protéine.

Les anticorps polyclonaux sont obtenus de façon tout à fait classique par injection à des lapins, comme cela sera détaillé ci-après. Toutefois, l'anticorps obtenu est nouveau puisqu'il est spécifique du nouvel antigène précédemment préparé.

On donnera ci-après un exemple de préparation de ces anticorps polyclonaux.

### Exemple 3 : préparation d'anticorps polyclonaux spécifiques de l'antigène de l'exemple 2

Le protocole d'immunisation a été réalisé à partir de deux lapins femelles de race NZ "S.S.C." de kg qui ont été traités de la façon décrite ci-après.

Une émulsion a été préparée en mélangeant 1 mg par litre du conjugué haptène-protéine précédemment préparé dans l'exemple 2, avec 1 ml d'adjuvant complet de Freund. Chaque lapin femelle a reçu 10 injections de 100 µl de l'émulsion précitée, dans le cou et sur le dos. Quatre semaines après les premières injections, les lapins ont reçu un premier rappel, pratiqué dans des conditions identiques. Après un nouvel intervalle de 4 semaines, ils reçoivent un nouveau rappel. Celui-ci est pratiqué par injection intramusculaire, au niveau de la hanche, de 0,5 ml d'une émulsion formée de 1 mg/ml de conjugué haptène-protéine et de 1 ml d'adjuvant incomplet de Freund.

Deux semaines plus tard, on prélève 2 à 5 ml de sang au niveau de l'oreille du lapin. Le sérum recueilli permet d'effectuer les premiers tests. Enfin, quatre semaines après le second rappel, un troisième rappel est effectué par injection intramusculaire selon le même principe que celui du deuxième rappel. Deux semaines plus tard, les lapins sont sacrifiés et l'on recueille le maximum de leur sang dont le sérum contient les anticorps spécifiques de l'antigène précité.

Les anticorps monoclonaux peuvent être obtenus de façon classique en faisant fusionner des cellules de myélome d'un mammifère tel qu'une souris avec des cellules de rate provenant par exemple de la lignée des souris BALB/c, ces souris ayant été immunisées par l'antigène (V) constitué par le morpholino nucléoside modifié lié à la protéine porteuse, (R⁵ représentant une protéine). Ces anticorps sont spécifiques des nouveaux antigènes obtenus par le procédé selon l'invention.

On donnera ci-après un exemple de préparation de ces anticorps monoclonaux.

### Exemple 4 : préparation d'anticorps monoclonaux spécifiques de l'antigène de l'exemple 2 (2-(5-hydroxycytosin-1yl)-4-carboxyméthyl-6-hydroxyméthylmorpholine)

### Immunisation :

Une suspension de l'antigène de l'exemple 2 précité a été émulsionnée dans un volume identique d'adjuvant complet de Freund. Cette émulsion a été injectée par voie intra-péritonéale à des souris femelles de la lignée BALB/c, agées de 6 à 8 semaines, à raison d'une dose de 0,1 ml d'émulsion. Trois semaines plus tard, une injection de rappel par voie intra-péritonéale est effectuée avec une émulsion de l'antigène de l'exemple 2 et d'adjuvant incomplet de Freund. Deux semaines après cette injection de rappel, l'immunisation finale est réalisée par une injection dans les veines de la queue de la souris, cette injection comprenant l'antigène ce l'exemple 2 dissous dans du NaCl 0,15 M

### Fusion cellulaire et clonage :

Trois jours après la dernière injection de rappel, les rates ont été prélevées sur les souris et broyées. Les cellules de rate ont été lavées dans du milieu de Dulbecco modifié ne contenant pas de sérum (DMEM). 10⁸ cellules de rates ont été mélangées à 10⁷ cellules de myélome puis centrifugées à 500xg pendant 7 minutes à température ambiante. On a éliminé le milieu et récupéré le culot de centrifugation auquel on a ajouté 0,8 ml de PEG 4000 à 50% (Merck), sur une période de 1 minutes avec agitation douce à 37°C. On y a ensuite ajouté du milieu de Dulbecco modifié, à raison de 1 ml pendant 1 minute, puis de 20 ml pendant 5 minutes. Les cellules ont été centrifugées à 200xg pendant 10 minutes puis le culot de centrifugation a été remis en suspension dans 15 ml de DMEM et 10% de FBS (sérum foetal de bovin). Des aliquots de 0,05 ml ont été distribués dans des plaques munies de puits de culture revêtus de macrophages. On a laissé incuber les plaques pendant 24 heures à 37°C avant d'ajouter dans les puits de l'hypoxanthine (1.10⁻⁴ M) de l'améthoptérine (4.10⁻⁷ M) et de la thymidine (1,6.10⁻⁵ M)) dans du DMEM (milieu HAT). Sept jours après la fusion, on a ajouté 0,025 ml de milieu HAT dans chaque puits et on a ensuite renouvelé le milieu tous les 3 à 4 jours. Les colonies ont été testées par la méthode ELISA pour leur activité à l'encontre de l'antigène de l'exemple 2. Seules, les cellules correspondant aux puits positifs ont été clonées.

## Revendications

1. Dérivés de nucléosides caractérisés en ce qu'ils répondent à la formule chimique suivante : 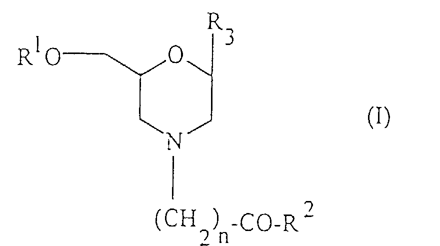 dans laquelle n est un nombre entier positif quelconque, R¹ représente H ou un acide mono, di ou tri-phosphorique linéaire, R² représente un groupe hydroxyle, un groupe alkyloxy, un groupe aryloxy, une protéine comprenant un site aminé libre, un aminoalkyl polystyrène ou une silice greffée avec une chaîne alkylamine et R³ représente une base nucléique modifiée choisie parmi l'uracile, la thymine, la cytosine, la guanine ou l'adénine modifiée.

2. Dérivés de nucléosides selon la revendication 1, caractérisés en ce que R³ est choisi parmi la (5-hydroxycytosin)-1yl, la (5-hydroxyhydantoïne)-1yl, -NH-CO-NH-CO-NH₂, le (5-hydroxyuracil)-1yl, le (5-formyluracil)-1yl, le (5-hydroxyméthyluracil)-1yl, -NH-CHO, la (5,6-dihydro-5,6-dihydroxythymin)-1yl, la (5,6-dihydrothymin)-1yl, la (5,6-dihydro-5-hydroxythymin)-1yl, l'adénine-N1-oxyde, la (8-oxo-7,8-dihydroadénine)-9yl, la [(6-amino-5-formylamino-pyrimidin)-4yl]amino, la (8-oxo-7,8-dihydroguanin)-9yl, la [(2-amino-6-oxo-5-formylamino-pyrimidin)-4yl]amino, la (4,8-dihydro-4-hydroxy-8-oxo-guanin)-9yl ou la [(2,2-diamino-oxazol-4one)-5yl]amino.

3. Dérivés de nucléosides selon la revendication 1 ou 2, caractérisé en ce que R² représente une protéine choisie parmi la sérumalbumine de bovin méthylée, l'ovalbumine de dinde ou de poulet ou les hémocyanines.

4. Procédé de fabrication des dérivés de nucléosides répondant à la formule chimique suivante : 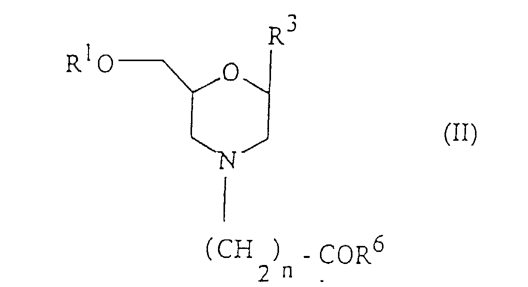 dans laquelle n est un nombre entier positif quelconque, R¹ représente H ou acide mono, di ou tri-phosphorique linéaire, R⁶ représente un groupe hydroxyle, un groupe alkyloxy ou un groupe aryloxy et R³ représente une base nucléique modifiée choisie parmi l'uracile, la thymine, la cytosine, la guanine ou l'adénine modifiée, consistant à modifier un dérivé de nucléoside répondant à la formule chimique suivante : 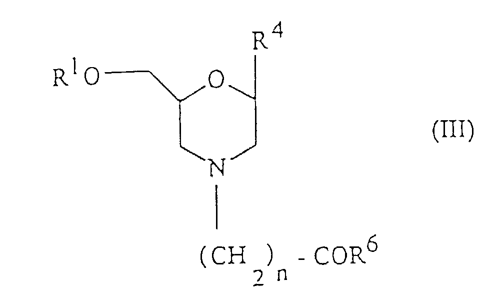 dans laquelle n, R¹ et R⁶ ont la même signification que précédemment et R⁴ représente une base choisie parmi l'uracile, la thymine, la cytosine, la guanine ou l'adénine, au moyen d'un agent substituant, dans lequel l'agent substituant est un agent chimique choisi parmi l'ozone, le peroxyde d'hydrogène, le brome combiné à la collidine, le brome et Ag₂O, le permanganate de potassium, le tétraoxyde d'osmium, le méthanal ou AlLiH₄.

5. Procédé de fabrication des dérivés de nucléosides répondant à la formule chimique suivante : 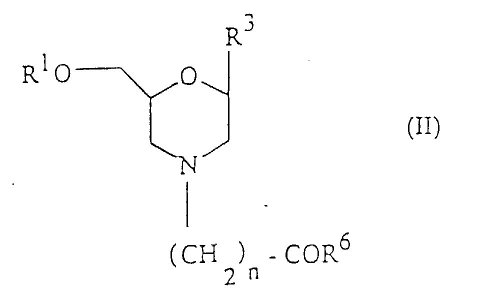 dans laquelle n est un nombre entier positif quelconque, R¹ représente H ou un acide mono, di ou tri-phosphorique linéaire, R⁶ représente un groupe hydroxyle, un groupe alkyloxy ou un groupe aryloxy et R³ représente une base nucléique modifiée choisie parmi l'uracile, la thymine, la cytosine, la guanine ou l'adénine modifiée, consistant à modifier un dérivé de nucléoside répondant à la formule chimique suivante : 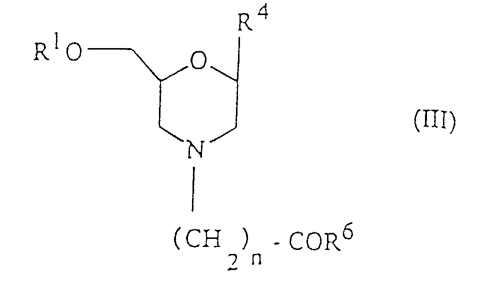 dans laquelle n, R¹ et R⁶ ont la même signification que précédemment et R⁴ représente une base choisie parmi l'uracile, la thymine, la cytosine, la guanine ou l'adénine, au moyen d'un agent substituant, dans lequel l'agent substituant est un traitement physique choisi parmi la photochimie, les rayonnements ionisants en présente ou en absence d'oxygène, l'hydrogénation catalytique ou le traitement par le brome et l'eau, puis l'hydrogénolyse.

6. Procédé de fabrication selon la revendication 4 ou 5, caractérisé en ce que R³ est choisi parmi la (5-hydroxycytosin)-1yl, la (5-hydroxyhydantoïne)-1yl, -NH-CO-NH-CO-NH₂, le (5-hydroxyuracil)-1yl, le (5-formyluracil)-1yl, le (5-hydroxyméthyluracil)-1yl, -NH-CHO, la (5,6-dihydro-5,6-dihydroxythymin)-1yl, la (5,6-dihydrothymin)-1yl, la (5,6-dihydro-5-hydroxythymin)-1yl, l'adénine-N1-oxyde, la (8--oxo-7,8-dihydroadénine)-9yl, la [(6-amino-5-formylaminopyrimidin)-4yl]amino, la (8-oxo-7,8-dihydroguanin)-9yl, la [(2-amino-6-oxo-5-formylamino-pyrimidin)-4yl]amino, la (4,8-dihydro-4-hydroxy-8-oxo-guanin)-9yl ou la [(2,2-diamino-oxazol-4one)-5yl]amino.

7. Procédé de fabrication d'un dérivé de nucléoside répondant à la formule chimique suivante : 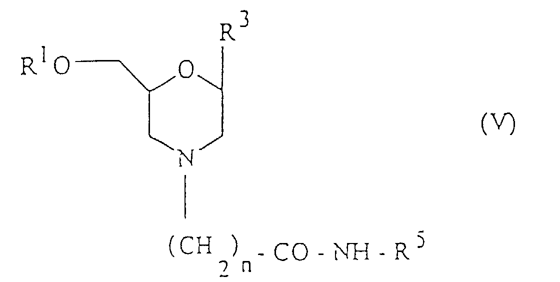 dans laquelle n est un nombre entier positif quelconque, R¹ représente H ou un acide mono, di ou triphosphorique linéaire, R⁵ représente le reste d'une protéine, un alkyl polystyrène ou une silice greffée avec une chaîne alkyle et R³ représente une base nucléique modifiée choisie parmi l'uracile, la thymine, la cytosine, la guanine ou l'adénine modifiée,
ce procédé consistant à faire réagir un composé du type NH₂-R⁵, dans lequel R⁵ a la même signification que précédemment, avec un dérivé de nucléoside répondant à la formule chimique suivante : 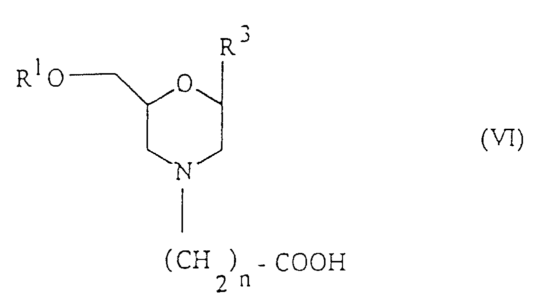 dans laquelle n, R¹ et R³ ont la même signification que précédemment.

8. Procédé de fabrication selon la revendication 7, caractérisé en ce que R³ est choisi parmi la (5-hydroxycytosin)-1yl, la (5-hydroxyhydantoïne)-1yl, -NH-CO-NH-CO-NH₂, le (5-hydroxyuracil)-1yl, le (5-formyluracil)-1yl, le (5-hydroxyméthyluracil)-1yl, -NH-CHO, la (5,6-dihydro-5,6-dihydroxythymin)-1yl, la (5,6-dihydrothymin)-1yl, la (5,6-dihydro-5-hydroxythymin)-1yl, l'adénine-N1-oxyde, la (8--oxo-7,8-dihydroadénine)-9yl, la [(6-amino-5-formylaminopyrimidin)-4yl]amino, la (8-oxo-7,8-dihydroguanin)-9yl, la [(2-amino-6-oxo-5-formylamino-pyrimidin)-4yl]amino, la (4,8-dihydro-4-hydroxy-8-oxo-guanin)-9yl ou la [(2,2-diamino-oxazol-4one)-5yl]amino.

9. Anticorps polyclonaux anti-dérivé de nucléoside, caractérisés en ce qu'ils sont obtenus par immunisation d'un mammifère approprié avec l'antigène répondant à la formule chimique suivante : 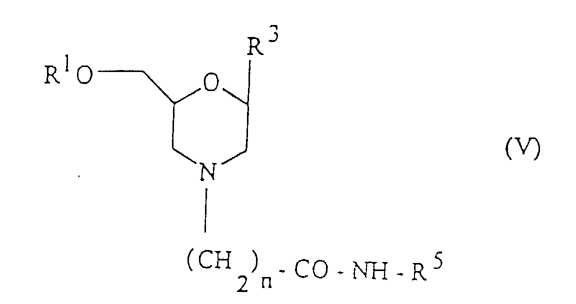 dans laquelle n est un nombre entier positif quelconque, R¹ représente H ou un acide mono, di ou triphosphorique linéaire, R³ représente une base nucléique modifiée choisie parmi l'uracile, la thymine, la cytosine, la guanine ou l'adénine modifiée, et R⁵ représente une protéine ne provenant pas dudit mammifère.

10. Anticorps polyclonaux selon la revendication 9, caractérisés en ce que R⁵ représente une protéine choisie parmi la sérumalbumine de bovin méthylée, l'ovalbumine de dinde ou de poulet ou les hémotcyanine.

11. Anticorps polyclonaux selon la revendication 9, caractérisés en ce que R³ est choisi parmi la (5-hydroxycytosin)-1yl, la (5-hydroxyhydantoïne)-1yl, -NH-CO-NH-CO-NH₂, le (5-hydroxyuracil)-1yl, le (5-formyluracil)-1yl, le (5-hydroxyméthyluracil)-1yl, -NH-CHO, la (5,6-dihydro-5,6-dihydroxythymin)-1yl, la (5,6-dihydrothymin)-1yl, la (5,6-dihydro-5-hydroxythymin)-1yl, l'adénine-N1-oxyde, la (8--oxo-7,8-dihydroadénine)-9yl, la [(6-amino-5-formylaminopyrimidin)-4yl]amino, la (8-oxo-7,8-dihydroguanin)-9yl, la [(2-amino-6-oxo-5-formylamino-pyrimidin)-4yl]amino, la (4,8-dihydro-4-hydroxy-8-oxo-guanin)-9yl ou la [(2,2-diamino-oxazol-4one)-5yl]amino.

12. Anticorps monoclonaux anti-dérivés de nucléosides, caractérisés en ce qu'ils sont obtenus en faisant fusionner des cellules de myélome d'un mammifère avec des cellules spléniques de souris immunisées par un antigène répondant à la formule chimique suivante : 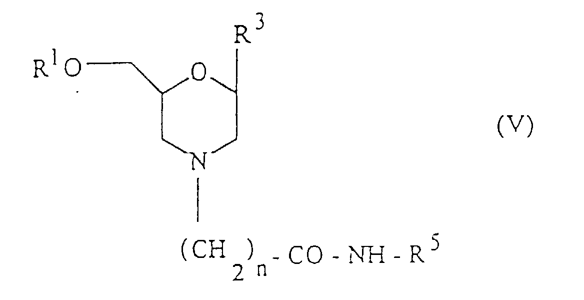 dans laquelle n représente un nombre entier positif quelconque, R¹ représente H ou un acide mono, di ou triphosphorique linéaire, R³ représente une base nucléique modifiée choisie parmi l'uracile, la thymine, la cytosine, la guanine ou l'adénine modifiée, et R⁵ représente une protéine ne provenant pas de la souris.

13. Anticorps monoclonaux selon la revendication 12, caractérisés en ce que R⁵ représente une protéine choisie parmi la sérumalbumine de bovin méthylée, l'ovalbumine de dinde ou de poulet ou les hémocyanines.

14. Anticorps monoclonaux selon la revendication 12, caractérisés en ce que R³ est choisi parmi la (5-hydroxycytosin)-1yl, la (5-hydroxyhydantoïne)-1yl, -NH-CO-NH-CO-NH₂, le (5-hydroxyuracil)-1yl, le (5-formyluracil)-1yl, le (5-hydroxyméthyluracil)-1yl, -NH-CHO, la (5,6-dihydro-5,6-dihydroxythymin)-1yl, la (5,6-dihydrothymin)-1yl, la (5,6-dihydro-5-hydroxythymin)-1yl, l'adénine-N1-oxyde, la (8-oxo-7,8-dihydroadénine)-9yl, la [(6-amino-5-formylamino-pyrimidin)-4yl]amino, la (8-oxo-7,8-dihydroguanin)-9yl, la [(2-amino-6-oxo-5-formylamino-pyrimidin)-4yl]amino, la (4,8-dihydro-4-hydroxy-8-oxo-guanin)-9yl ou la [(2,2-diamino-oxazol-4one)-5yl]amino.

## Patentansprüche

1. Nucleosid-Derivate, dadurch gekennzeichnet, daß sie der folgenden chemischen Formel entsprechen: 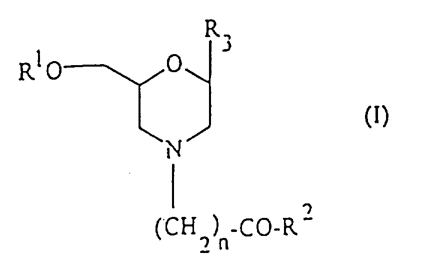 worin n eine beliebige positive ganze Zahl ist, R¹ H oder eine *lineare Mono-*, *Di-* oder Triphosphorsäure darstellt, R² eine Hydroxylgruppe, eine Alkyloxygruppe, eine Aryloxygruppe, ein Protein, das eine freie Aminostelle enthält, ein Aminoalkylpolystyrol oder ein mit einer Alkylamin-Kette gepfropftes Siliciumdioxid darstellt und R³ eine unter modifiziertem Uracil, Thymin, Cytosin, Guanin oder Adenin gewählte modifizierte Nucleinbase darstellt.

2. Nucleosid-Derivate nach Anspruch 1, dadurch gekennzeichnet, daß R³ gewählt ist unter (5-Hydroxycytosin)-1-yl, (5-Hydroxyhydantoin)-1-yl, -NH-CO-NH-CO-NH₂, (5-Hydroxyuracil)-1-yl, (5-Formyluracil)-1-yl, (5-Hydroxymethyluracil)-1-yl, -NH-CHO, (5,6-Dihydro-5,6-dihydroxythymin)-1-yl, (5,6-Dihydrothymin)-1-yl, (5,6-Dihydro-5-hydroxythymin)-1-yl, Adenin-N1-oxid, (8-Oxo-7,8-dihydroadenin)-9-yl, [(6-Amino-5-formylamino-pyrimidin)-4-yl] amino, (8-Oxo-7,8-dihydroguanin)-9-yl, [(2-Amino-6-oxo-5-formylamino-pyrimidin)-4-yl]amino, (4,8-Dihydro-4-hydroxy-8-oxo-guanin)-9-yl oder [(2,2-Diaminooxazol-4-on)-5-yl]amino.

3. Nucleosid-Derivate nach Anspruch 1 oder 2, dadurch gekennzeichnet, daß R² ein unter dem methylierten Rinderserumalbumin, dem Puten- oder Hühner-Ovalbumin oder den Hämocyaninen gewähltes Protein darstellt.

4. Verfahren zur Herstellung der Nucleosid-Derivate entsprechend der folgenden chemischen Formel: 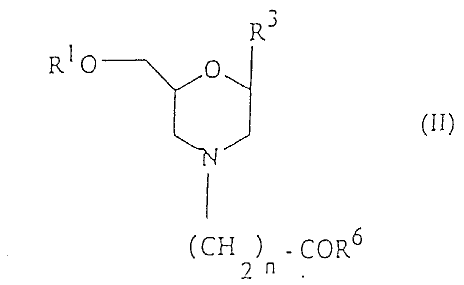 worin n eine beliebige positive ganze Zahl ist, R¹ H oder eine lineare Mono-, Di- oder Triphosphorsäure darstellt, R⁶ eine Hydroxylgruppe, eine Alkyloxygruppe oder eine Aryloxygruppe darstellt und R³ eine unter modifiziertem Uracil, Thymin, Cytosin, Guanin oder Adenin gewählte modifizierte Nucleinbase darstellt, darin bestehend, ein Nucleosid-Derivat, das der folgenden chemischen Formel entspricht: 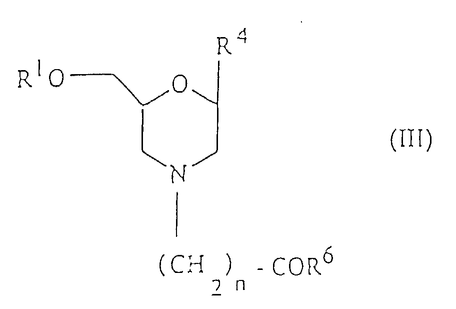 worin n, R¹ und R⁶ die gleiche Bedeutung haben wie vorher und R⁴ eine unter Uracil, Thymin, Cytosin, Guanin oder Adenin gewählte Base darstellt, mit Hilfe eines substituierenden Reagens zu modifizieren, wobei das substituierende Reagens ein chemisches Reagens ist, gewählt unter Ozon, Wasserstoffperoxid, Brom kombiniert mit Collidin, Brom und Ag₂O, Kaliumpermanganat, Osmiumtetroxid, Formaldehyd oder LiAlH₄.

5. Verfahren zur Herstellung der Nucleosid-Derivate entsprechend der folgenden chemischen Formel: 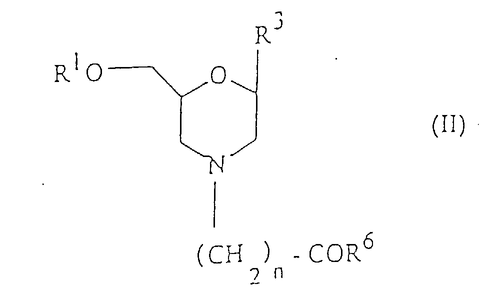 worin n eine beliebige positive ganze Zahl ist, R¹ H oder eine lineare Mono-, Di- oder Triphosphorsäure darstellt, R⁶ eine Hydroxylgruppe, eine Alkyloxygruppe oder eine Aryloxygruppe darstellt und R³ eine unter modifiziertem Uracil, Thymin, Cytosin, Guanin oder Adenin gewählte modifizierte Nucleinbase darstellt, darin bestehend, ein Nucleosid-Derivat, das der folgenden chemischen Formel entspricht: 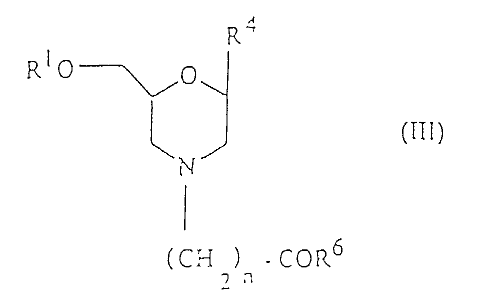 worin n, R¹ und R⁶ die gleiche Bedeutung haben wie vorher und R⁴ eine unter Uracil, Thymin, Cytosin, Guanin oder Adenin gewählte Base darstellt, mit Hilfe eines substituierenden Reagens zu modifizieren, wobei das substituierende Reagens eine physikalische Behandlung ist, gewählt unter der Photochemie, den ionisierenden Strahlungen in Gegenwart oder Abwesenheit von Sauerstoff, der katalytischen Hydrierung oder der Behandlung mit Brom und Wasser mit nachfolgender Hydrogenolyse.

6. Herstellungsverfahren nach Anspruch 4 oder 5, dadurch gekennzeichnet, daß R³ gewählt ist unter (5-Hydroxycytosin)-1-yl, (5-Hydroxyhydantoin)-1-yl, -NH-CO-NH-CO-NH₂, (5-Hydroxyuracil)-1-yl, (5-Formyluracil)-1-yl, (5-Hydroxymethyluracil)-1-yl, -NH-CHO, (5,6-Dihydro-5,6-dihydroxythymin)-1-yl, (5,6-Dihydrothymin)-1-yl, (5,6-Dihydro-5-hydroxythymin)-1-yl, Adenin-N1-oxid, (8-Oxo-7,8-dihydroadenin)-9-yl, [(6-Amino-5-formylamino-pyrimidin)-4-yl]amino, (8-Oxo-7,8-dihydroguanin)-9-yl, [(2-Amino-6-oxo-5-formylaminopyrimidin)-4-yl]amino, (4,8-Dihydro-4-hydroxy-8-oxo-guanin)-9-yl oder [(2,2-Diamino-oxazol-4-on)-5-yl]amino.

7. Verfahren zur Herstellung eines Nucleosid-Derivats entsprechend der folgenden chemischen Formel: 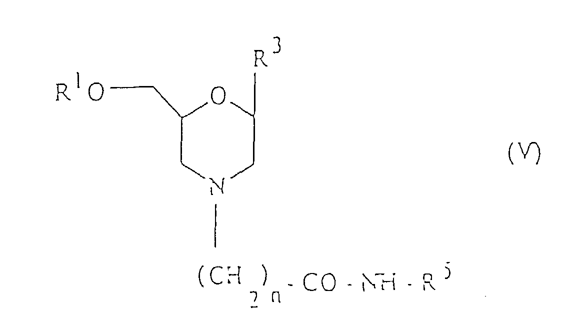 worin n eine beliebige positive ganze Zahl ist, R¹ H oder eine lineare Mono-, Di- oder Triphosphorsäure darstellt, R⁵ einen Proteinrest, ein Alkylpolystyrol oder ein mit einer Alkylkette gepfropftes Siliciumdioxid darstellt und R³ eine unter modifiziertem Uracil, Thymin, Cytosin, Guanin oder Adenin gewählte modifizierte Nucleinbase darstellt, wobei dieses Verfahren darin besteht, eine Verbindung des Typs NH₂-R⁵, in der R⁵ die gleiche Bedeutung hat wie vorher, mit einem Nucleosid-Derivat reagieren zu lassen, das der folgenden chemischen Formel entspricht: 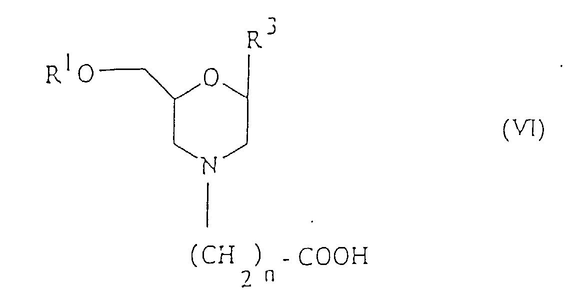 worin n, R¹ und R³ die gleiche Bedeutung wie vorher haben.

8. Herstellungsverfahren nach Anspruch 7, dadurch gekennzeichnet, daß R³ gewählt ist unter (5-Hydroxycytosin)-1-yl, (5-Hydroxyhydantoin)-1-yl, -NH-CO-NH-CO-NH₂, (5-Hydroxyuracil)-1-yl, (5-Formyluracil)-1-yl, (5-Hydroxymethyluracil)-1-yl, -NH-CHO, (5,6-Dihydro-5,6-dihydroxythymin)-1-yl, (5,6-Dihydrothymin)-1-yl, (5,6-Dihydro-5-hydroxythymin)-1-yl, Adenin-N1-oxid, (8-Oxo-7,8-dihydroadenin)-9-yl, [(6-Amino-5-formylamino-pyrimidin)-4-yl]amino, (8-Oxo-7,8-dihydroguanin)-9-yl, [(2-Amino-6-oxo-5-formylamino-pyrimidin)-4-yl]amino, (4,8-Dihydro-4-hydroxy-8-oxo-guanin)-9-yl oder [(2,2-Diaminooxazol-4-on)-5-yl]amino.

9. Polyklonale Antikörper, Nucleosid-Anti-Derivate, dadurch gekennzeichnet, daß sie erhalten werden durch Immunisierung eines geeigneten Säugetiers mit dem Antigen, das der folgenden chemischen Formel entspricht: 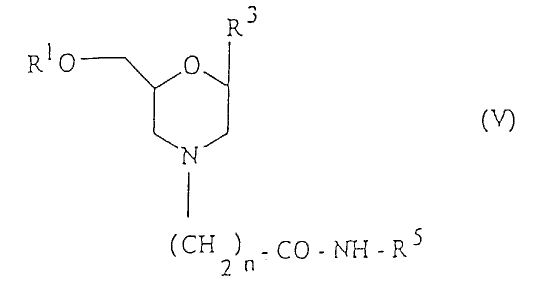 worin n eine beliebige positive ganze Zahl ist, R¹ H oder eine lineare Mono-, Di- oder Triphosphorsäure darstellt, R³ eine unter modifiziertem Uracil, Thymin, Cytosin, Guanin oder Adenin gewählte modifizierte Nucleinbase darstellt und R⁵ ein Protein darstellt, das nicht von dem genannten Säugetier stammt.

10. Polyklonale Antikörper nach Anspruch 9, dadurch gekennzeichnet, daß R⁵ ein unter dem methylierten Rinder-Serumalbumin, dem Puten- oder Hühner-Ovalbumin oder den Hämocyaninen gewähltes Protein darstellt.

11. Polyklonale Antikörper nach Anspruch 9, dadurch gekennzeichnet, daß R³ gewählt ist unter (5-Hydroxycytosin)-1-yl, (5-Hydroxyhydantoin)-1-yl, -NH-CO-NH-CO-NH₂, (5-Hydroxyuracil)-1-yl, (5-Formyluracil)-1-yl, (5-Hydroxymethyluracil)-1-yl, -NH-CHO, (5,6-Dihydro-5,6-dihydroxythymin)-1-yl, (5,6-Dihydrothymin)-1-yl, (5,6-Dihydro-5-hydroxythymin)-1-yl, Adenin-N1-oxid, (8-Oxo-7,8-dihydroadenin)-9-yl, [(6-Amino-5-formylamino-pyrimidin)-4-yl]amino, (8-Oxo-7,8-dihydroguanin)-9-yl, [(2-Amino-6-oxo-5-formylamino-pyrimidin)-4-yl]amino, (4,8-Dihydro-4-hydroxy-8-oxo-guanin)-9-yl oder [(2,2-Diaminooxazol-4-on)-5-yl]amino.

12. Monoklonale Antikörper, Nucleosid-Anti-Derivate, dadurch gekennzeichnet, daß sie erhalten werden, indem man Myelomzellen eines Säugetiers verschmelzen läßt mit Milzzellen von Mäusen, die durch ein Antigen immunisiert sind, das der folgenden chemischen Formel entspricht: 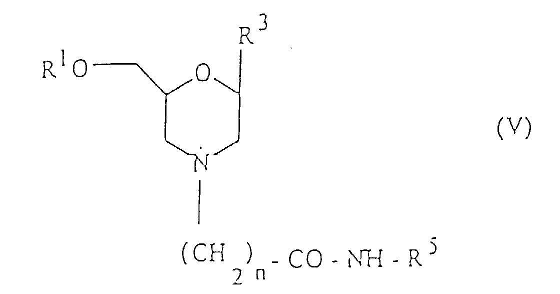 worin n eine beliebige positive ganze Zahl darstellt, R¹ H oder eine lineare Mono-, Di- oder Triphosphorsäure darstellt, R³ eine unter modifiziertem Uracil, Thymin, Cytosin, Guanin oder Adenin gewählte modifizierte Nucleinbase darstellt und R⁵ ein Protein darstellt, das nicht von der Maus stammt.

13. Monoklonale Antikörper nach Anspruch 12, dadurch gekennzeichnet, daß R⁵ ein unter dem methylierten Rinder-Serumalbumin, dem Puten- oder Hühner-Ovalbumin oder den Hämocyaninen gewähltes Protein darstellt.

14. Monoklonale Antikörper nach Anspruch 12, dadurch gekennzeichnet, daß R³ gewählt ist unter (5-Hydroxycytosin)-1-yl, (5-Hydroxyhydantoin)-1-yl, -NH-CO-NH-CO-NH₂, (5-Hydroxyuracil)-1-yl, (5-Formyluracil)-1-yl, (5-Hydroxymethyluracil)-1-yl, -NH-CHO, (5,6-Dihydro-5,6-dihydroxythymin)-1-yl, (5,6-Dihydrothymin)-1-yl, (5,6-Dihydro-5-hydroxythymin)-1-yl, Adenin-N1-oxid, (8-Oxo-7,8-dihydroadenin)-9-yl, [(6-Amino-5-formylamino-pyrimidin)-4-yl]amino, (8-Oxo-7,8-dihydroguanin)-9-yl, [(2-Amino-6-oxo-5-formylamino-pyrimidin)-4-yl]amino, (4,8-Dihydro-4-hydroxy-8-oxo-guanin)-9-yl oder [(2,2-Diaminooxazol-4-on)-5-yl]amino.

## Claims

1. Derivative of nucleosides, characterized in that they comply with the following chemical formula: 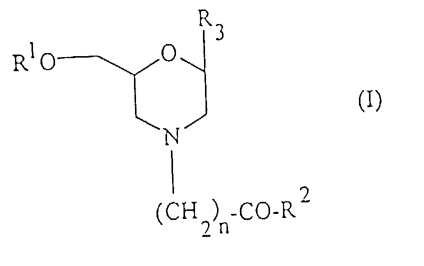 in which n is a random, positive integer, R¹ stands for H or a linear mono-, di or tri-phosphoric acid, R² stands for a hydroxyl group, an alkyloxy group, an aryloxy group, a protein incorporating a free amino site, an aminoalkyl polystyrene or a silica grafted to an alkyl amine chain and R³ stands for a modified, nucleic base chosen from among uracil, thymine, cytosine, guanine or adenine in modified form.

2. Derivatives of nucleosides according to claim 1, characterized in that R³ is chosen from among (5-hydroxycytosin)-1-yl, (5-hydroxyhydantoin)-1-yl, -NH-CO-NH-CO-NH₂, (5-hydroxyuracil)-1-yl, (5-formyluracil)-1-yl, (5-hydroxymethyluracil)-1-yl, -NH-CHO, (5,6-dihydroxythymin)-1-yl, (5,6-dihydrothymin)-1-yl, (5,6-dihydro-5-hydroxythymin)-1-yl, adenine-N1-oxide, (8-oxo-7,8-dihydroadenin)-9-yl, [(6-amino-5-formylamino-pyrimidin)-4-yl]amino, (8-oxo-7,8-dihydroguanin)-9-yl, [(2-amino-6-oxo-5-formylamino-pyrimidin)-4-yl] amino, (4,8-dihydro-4-hydroxy-8-oxo-guanin)-9-yl, [(2,2-diamino-oxazol-4one)-5-yl]amino.

3. Derivatives of nucleosides according to claim 1 or 2, characterized in that R² stands for a protein chosen from among methylated bovine serum albumin, turkey or chicken egg albumin or hemocyanins.

4. Process for the production of derivatives of nucleosides in accordance with the following chemical formula: 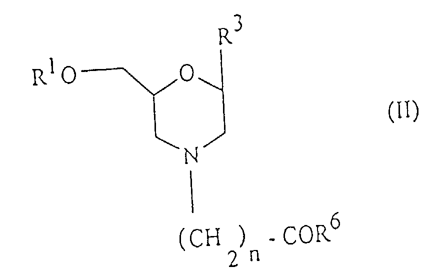 in which n is a random, positive integer, R¹ stands for H or a linear mono-, di- or tri-phosphoric acid, R⁶ stands for a hydroxyl group, an alkyloxy group or an aryloxy group and R³ stands for a modified, nucleic base chosen from among uracil, thymine, cytosine, guanine or adenine in modified form consisting of reacting a substituent with a nucleoside derivative complying with the following chemical formula: 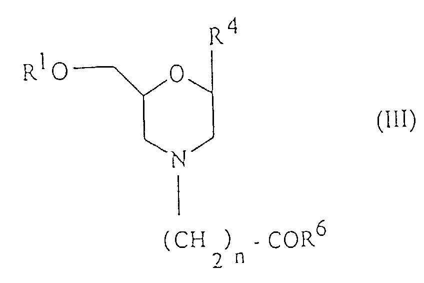 in which n, R¹ and R⁶ have the same meanings as hereinbefore and R⁴ stands for a base chosen from among uracil, thymine, cytosine, guanine or adenine, by means of a substituent, in which the substituent is a chemical agent chosen from among ozone, hydrogen peroxide, bromine combined with collidine, bromine and Ag₂O, potassium permanganate, osmium tetraoxide, methanal or AlLiH₄.

5. Process for the production of derivatives of nucleosides in accordance with the following chemical formula: 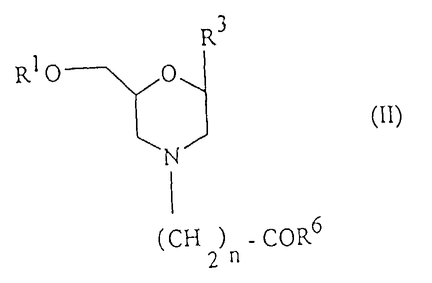 in which n is a random, positive integer, R¹ stands for H or a linear mono-, di or tri-phosphoric acid, R⁶ stands for a hydroxyl group, an alkyloxy group or an aryloxy group and R³ stands for a modified, nucleic base chosen from among uracil, thymine, cytosine, guanine or adenine in modified form consisting of reacting a substituent with a nucleoside derivative complying with the following chemical formula: 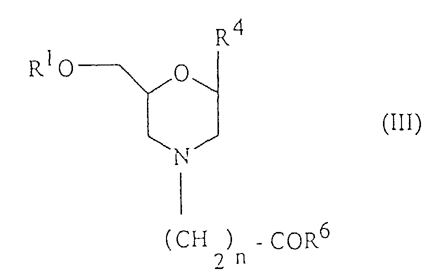 in which n, R¹ and R⁶ have the same meanings as hereinbefore and R⁴ stands for a base chosen from among uracil, thymine, cytosine, guanine or adenine, by means of a substituent, in which the substituent is a physical treatment chosen from among photochemistry, ionizing rays in the presence or absence of oxygen, catalytic hydrogenation or treatment by bromine and water, followed by hydrogenolysis.

6. Production process according to claim 4 or 5, characterized in that R³ is chosen from among (5-hydroxycytosin)-1-yl, (5-hydroxyhydantoin)-1-yl, -NH-CO-NH-CO-NH₂, (5-hydroxyuracil)-1-yl, (5-formyluracil)-1-yl, (5-hydroxy-methyluracil)-1-yl, -NH-CHO, (5,6-dihydro-5,6-dihydroxythymin)-1-yl, (5,6-dihydrothymin)-1-yl, (5,6-dihydro-5-hydroxythymin)-1-yl, adenine-N1-oxide, (8-oxo-7,8-dihydroadenine)-9-yl, [(6-amino-5-formylamino-pyrimidin)-4-yl]-amino, (8-oxo-7,8-dihydroguanin)-9-yl, [(2-amino-6-oxo-5-formylaminopyrimidin)-4-1]-amino, (4,8-dihydro-4-hydroxy-8-oxo-guanin)-9-yl or [(2,2-diamino-oxazol-4-one)-5-yl]-amino.

7. Process for the production of a nucleoside derivative complying with the following chemical formula: 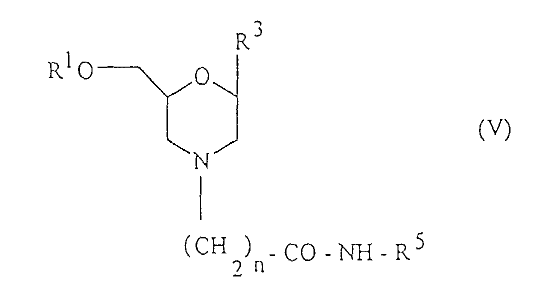 in which n is a random, positive integer, R¹ stands for H or a linear mono-, di- or tri-phosphoric acid, R⁵ stands for the residue of a protein, an alkyl polystyrene or a silica grafted to an alkyl chain and R³ stands for a modified, nucleic base chosen from among uracil, thymine, cytosine, guanine or adenine in modified form, said process consisting of reacting a compound of type NH₂-R⁵, in which R⁵ has the same meaning as hereinbefore, with a nucleoside derivative complying with the following chemical formula: 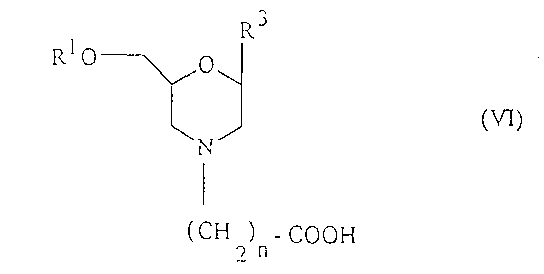 in which n, R¹ and R³ have the same meanings as hereinbefore.

8. Production process according to claim 7, characterized in that R³ is chosen from among (5-hydroxycytosin)-1-yl, (5-hydroxyhydantoin)-1-yl, -NH-CO-NH-CO-NH₂, (5-hydroxyuracil)-1-yl, (5-formyluracil)-1-yl, (5-hydroxymethyluracil)-1-yl, -NH-CHO, (5,6-dihydro-5,6-dihydroxythymin)-1-yl, (5,6-dihydrothymin)-1-yl, (5,6-dihydro-5-hydroxythymin)-1-yl, adenine-N1-oxide, (8-oxo-7,8-dihydroadenine)-9-yl, [(6-amino-5-formylamino-pyrimidin)-4-yl], amino, (8-oxo-7,8-dihydroguanin)-9-yl, [(2-amino-6-oxo-5-formylaminopyrimidin)-4-yl]-amino, (4,8-dihydro-4-hydroxy-8-oxo-guanin)-9-yl or [(2,2-diamino-oxazol-4-one)-5-yl]-amino.

9. Polyclonal antibodies anti-derived from nucleoside, characterized in that they are obtained by the immunization of an appropriate mammal with the antigen complying with the following chemical formula: 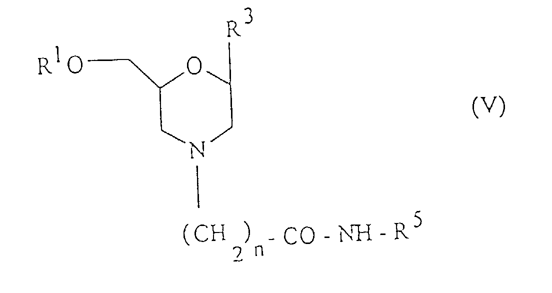 in which n is a random, positive integer, R¹ stands for H or a linear mono-, di- or tri-phosphoric acid, R³ stands for a modified, nucleic base chosen from among uracil, thymine, cytosine, guanine or adenine in modified form and R⁵ stands for a protein not from said mammal.

10. Polyclonal antibodies according to claim 9, characterized in that R⁵ represents a protein chosen from among methylated bovine serum albumin, turkey or chicken egg albumin in hemocyanins.

11. Polyclonal antibodies according to claim 9, characterized in that R³ is chosen from among (5-hydroxycytosin)-1-yl, (5-hydroxyhydantoin)-1-yl, -NH-CO-NH-CO-NH₂, (5-hydroxyuracil)-1-yl, (5-formyluracil)-1-yl, (5-hydroxymethyluracil)-1-yl, -NH-CHO, (5,6-dihydro-5,6-dihydroxythymin)-1-yl, (5,6-dihydrothymin)-1-yl, (5,6-dihydro-5-hydroxythymin)-1-yl, adenine-N1-oxide, (8-oxo-7,8-dihydroadenine)-9-yl, [(6-amino-5-formylamino-pyrimidin)-4-yl]-amino, (8-oxo-7,8-dihydroguanin)-9-yl, [(2-amino-6-oxo-5-formylaminopyrimidin)-4-yl]-amino, (4,8-dihydro-4-hydroxy-8-oxo-guanin)-9-yl or [(2,2-diamino-oxazol-4-one)-5-yl]-amino.

12. Monoclonal antibodies anti-derived from nucleoside, characterized in that they are obtained by fusing myeloma cells of a mammal with spleen cells of mice immunized by an antigen complying with the following chemical formula: 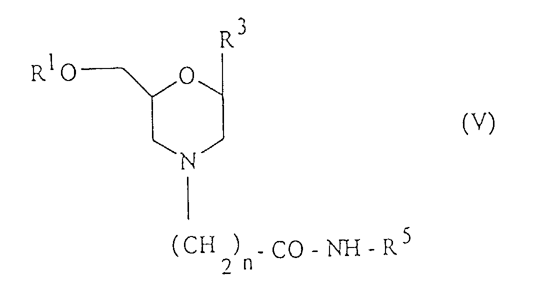 in which n represents a random, positive integer, R¹ stands for H or a linear mono-, di- or tri-phosphoric acid, R³ stands for a modified nucleic base chosen from among uracil, thymine, cytosine, guanine or adenine in modified form and R⁵ represents a protein not from the mouse.

13. Monoclonal antibodies according to claim 12, characterized in that R⁵ represents a protein chosen from among methylated bovine serum albumin, chicken or turkey egg albumin or hemocyanins.

14. Monoclonal antibodies according to claim 12, characterized in that R³ is chosen from among (5-hydroxycytosin)-1-yl, (5-hydroxyhydantoin)-1-yl, -NH-CO-NH-CO-NH₂, (5-hydroxyuracil)-1-yl, (5-formyluracil)-1-yl, (5-hydroxymethyluracil)-1-yl, -NH-CHO, (5,6-dihydro-5,6-dihydroxythymin)-1-yl, (5,6-dihydrothymin)-1-yl, (5,6-dihydro-5-hydroxythymin)-1-yl, adenine-N1-oxide, (8-oxo-7,8-dihydroadenine-9-yl, [(6-amino-5-formylamino-pyrimidin)-4-yl]amino, (8-oxo-7,8-dihydroguanin)-9-yl, [(2-amino-6-oxo-5-formylaminopyrimidin)-4-yl]-amino, (4,8-dihydro-4-hydroxy-8-oxo-guanin)-9-yl or [(2,2-diamino-oxazol-4-one)-5-yl]-amino.
